# FASCICULE DE BREVET EUROPEEN

(11) **EP 1 027 326 B1**
(45) Date de publication et mention de la délivrance du brevet: **15.06.2005**
(21) Numéro de dépôt: 98947631.2
(22) Date de dépôt: 08.10.1998
(51) Int. Cl.: C07C 231/12, C07C 309/66, C07C 303/30, C07C 45/56, C07C 45/46

(54) **PROCEDE DE FONCTIONNALISATION D'UN COMPOSE PHENOLIQUE PORTEUR D'UN GROUPE ELECTRO-DONNEUR**
VERFAHREN ZUR FUNKTIONALISIERUNG EINES MIT EINER ELEKTRONENDONOR-GRUPPE SUBSTITUIERTEN PHENOLS
METHOD FOR FUNCTIONALIZING A PHENOLIC COMPOUND BEARING AN ELECTRON-DONOR GROUP

(30) Priorité: 08.10.1997 FR 9712548
(43) Date de publication de la demande: 16.08.2000
(73) Titulaire: RHODIA CHIMIE, 92408 Courbevoie Cédex (FR)
(72) Inventeur: MIOKOWSKI, Charles, F-67200 Strasbourg (FR); WAGNER, Alain, F-67100 Strasbourg (FR); BENSEL, Nicolas, F-68120 Pfastatt (FR); PEVERE, Virginie, F-69008 Lyon (FR); DESMURS, Jean-Roger, F-69360 Saint Symphorien d'Ozon (FR)
(74) Mandataire: Dutruc-Rosset, Marie-Claude
(86) Numéro de dépôt international: PCT/FR1998/002155
(87) Numéro de publication internationale: WO 1999/018064

(56) Documents cités:
- FR-A- 2 446 273
- SCARPATIE ET AL.: "Selective formylation of diphenols" SYNTHETIC COMMUNICATIONS, vol. 20, no. 17, 1990, pages 2565-2572, XP002089516
- GREENE T. W.; WUTS P. : "PROTECTIVE GROUPS IN ORGANIC SYNTHESIS." 1991 , JOHN WILEY & SONS, INC , NEW YORK XP002089517 207350 voir page 168 - page 170
- BULLETIN DE LA SOCIETE CHIMIQUE DE FRANCE, no. 5-6, 1978, pages II-248-II-254, XP002067206 PARIS FR

## Description

La présente invention a pour objet un procédé de fonctionnalisation d'un composé phénolique porteur d'un groupe électro-donneur, en position para dudit groupe.

L'invention concerne entre autres, un procédé d'amidoalkylation d'un composé phénolique porteur d'un groupe électro-donneur.

L'invention vise plus particulièrement un composé phénolique porteur d'un groupe électro-donneur de préférence, en position ortho du groupe hydroxyle.

Dans l'exposé qui suit de la présente invention, on entend "par composé phénolique", tout composé aromatique porteur d'au moins un groupe hydroxyle et par "aromatique", la notion classique d'aromaticité telle que définie dans la littérature, notamment par Jerry MARCH, Advanced Organic Chemistry, 4^{ème} édition, John Wiley and Sons, 1992, pp. 40 et suivantes.

Le composé phénolique qui intervient dans le procédé de l'invention est un composé phénolique porteur un groupe électro-donneur, de préférence en position ortho du groupe hydroxyle et possède un atome d'hydrogène en position para du groupe électro-donneur.

Dans le présent texte, on entend par "groupe électro-donneur", un groupe tel que défini par H.C. BROWN dans l'ouvrage de Jerry MARCH - Advanced Organic Chemistry, 4^{ème} édition, John Wiley and Sons, 1992, chapitre 9, pp. 273 - 292.

M.L. Scarpati et al ont décrit (Synthetic Communications (1990), 20(7), 2565-2572) la formylation régiosélective de composés ortho- et para-diphénoliques en utilisant des groupes protecteurs tels que méthyle et méthoxycarbonyle.

L'amidoalkylation d'un composé phénolique porteur d'un groupe électro-donneur pose problème à l'Homme du Métier.

Ainsi, dans le cas plus particulier de l'amidométhylation du gaïacol (ou 2-méthoxyphénol), la réaction s'effectue plus particulièrement en position 4 par rapport au groupe hydroxyle.

L'objectif de la présente invention est de fournir un procédé permettant d'effectuer préférentiellement l'amidoalkylation et, plus particulièrement, l'amidométhylation en position 4 par rapport au groupe méthoxy.

Poursuivant ses recherches, la demanderesse a trouvé que le procédé pouvait s'appliquer non seulement pour les réactions d'amidoalkylation mais aussi pour toutes les réactions de substitution électrophile qui font intervenir un réactif électrophile qui comprend au moins un atome de carbone électrophile C⁺, c'est-à-dire un atome de carbone présentant un déficit électronique ou bien un atome de soufre électrophile.

L'objet de la présente invention est un procédé de fonctionnalisation en position para par rapport à un groupe électro-donneur porté par un composé phénolique caractérisé par le fait que l'on soumet le composé phénolique porteur d'un groupe électro-donneur aux étapes suivantes :
- une première étape qui consiste à effectuer la protection du groupe hydroxyle sous la forme d'une fonction ester sulfonique,
- une deuxième étape qui consiste à faire réagir le composé phénolique protégé avec un réactif électrophile,
- éventuellement une troisième étape de déprotection du groupe hydroxyle.

Il a été trouvé selon l'invention qu'il était possible d'introduire un groupement comprenant un atome de carbone ou de soufre électrophile en position para du groupe électro-donneur porté par un composé phénolique, dans la mesure où l'on protégeait le groupe hydroxyle par une fonction ester sulfonique.

Il est possible selon l'invention d'appliquer ce procédé à toutes les réactions de substitution électrophile et l'on peut citer notamment les réactions d'amidoalkylation, les réactions de Friedel-Crafts telles que notamment acylation, sulfonylation, alkylation, les réactions d'aminoalkylation, d'hydroxyalkylation, de formylation, de carboxylation etc...

### Protection du groupe hydroxyle.

Conformément au procédé de l'invention, on effectue dans une première étape, la protection du groupe hydroxyle du composé phénolique de départ.

On part d'un composé phénolique porteur d'au moins un groupe électro-donneur, de préférence, en position ortho par rapport au groupe hydroxyle et dont la position en para du groupe électro-donneur est libre de tout substituant.

Plus particulièrement, il répond à la formule générale (I) : 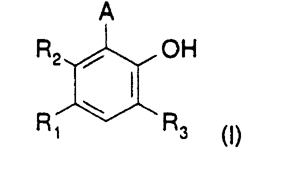 dans ladite formule (I) :
- A représente un groupe électro-donneur,
- R₁, R₂ et R₃, identiques ou différents, représentent :
   . un atome d'hydrogène,
   . un radical alkyle linéaire ou ramifié ayant de 1 à 20 atomes de carbone éventuellement porteur de un ou plusieurs atomes d'halogène,
   . un groupe cycloalkyle ayant de 3 à 8 atomes de carbone, et de préférence, 6 atome de carbone,
   . un radical aralkyle ayant de 6 à 20 atomes de carbone,
   . un radical aryle ayant de 6 à 20 atomes de carbone,
   . un atome d'halogène,
   . un groupe électro-attracteur,
- deux groupes R₁ et R₂ peuvent former avec les deux atomes qui les portent, un cycle carbocyclique, saturé, insaturé ou aromatique ayant de 3 à 8 atomes de carbone.

Dans la formule (I), les groupes R₁, R₂ et R₃ peuvent représenter un groupe électro-attracteur.

On entend par "groupe électro-attracteur", un groupe tel que défini par H.C. BROWN dans l'ouvrage de Jerry MARCH - Advanced Organic Chemistry, 4^{ème} édition, John Wiley and Sons, 1992, chapitre 9, pp. 273 - 292.

Il s'agit préférentiellement d'un groupe carboxylique ou ester (ayant de préférence, de 3 à 8 atomes de carbone) ou un groupe nitrile ou un groupe nitro.

Les composés mis en oeuvre préférentiellement, répondent à la formule (I) dans laquelle R₁, R₂ et R₃ représentent un atome d'hydrogène, un groupe alkyle linéaire ou ramifié ayant de 1 à 4 atomes de carbone, un atome d'halogène ou un groupe trifluorométhyle.

Parmi les composés de départ répondant à la formule (I), on choisit ceux de formule (I) dans laquelle A représente l'un des groupes ou fonctions suivantes :
- un groupe alkyle linéaire ou ramifié ayant de préférence de 1 à 6 atomes de carbone et encore plus préférentiellement de 1 à 4 atomes de carbone,
- un groupe cycloalkyle ayant de 3 à 8 atomes de carbone, et de préférence, 6 atome de carbone,
- le radical phényle,
- le radical benzyle ou phényléthyle,
- le groupe hydroxyle,
- un atome de fluor,
- un groupe alkoxy ayant de 1 à 6 atomes de carbone encore plus préférentiellement de 1 à 4 atomes de carbone dans la partie alkyle ou un groupe phénoxy,
- un groupe amino de préférence disubstitué dans lesquels les substituants identiques ou différents sont des radicaux alkyle linéaire ou ramifié ayant de 1 à 6 atomes de carbone et encore plus préférentiellement de 1 à 4 atomes de carbone ou phényle,
- un groupe alkylamide ou arylamide dans lequel le groupe alkyle a de 1 à 6 atomes de carbone et de préférence de 1 à 4 atomes de carbone et le groupe aryle a de 6 à 12 atomes de carbone et est de préférence, un groupe phényle.

Les composés mis en oeuvre préférentiellement, répondent à la formule (I) dans laquelle A représente un groupe méthoxy ou éthoxy.

Le procédé de l'invention consiste dans une première étape à protéger la fonction hydroxyle par un groupement protecteur. A cet effet, on transforme la fonction hydroxyle en ester sulfonique.

Les groupes ester sulfonique préférés sont :
- les tosylates (p-toluènesulfonates) -OSO₂-C₆H₄-CH₃
- les brosylates (p-bromobenzènesulfonates) -OSO₂-C₆H₄-Br
- les nosylates (p-nitrobenzènesulfonates) -OSO₂-C₆H₄-NO₂
- les mésylates (méthanesulfonates) -OSO₂-CH₃
- les bétylates (ammonioalcanesulfonates) -OSO₂-(CH₂)ₙNMe₃⁺ avec n compris entre 0 et 6,
- les triflates (trifluorométhanesulfonates) -OSO₂-CF₃
- les nonaflates (nonafluorobutanesulfonates) -OSO₂-C₄F₉
- les trésylates (2,2,2-trifluroéthanesulfonates).-OSO₂-CH₂-CF₃

Ainsi, le composé phénolique, sous forme protégée répond à la formule générale (II) : 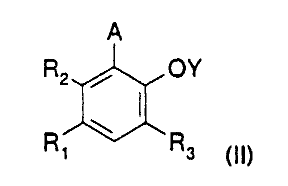 dans ladite formule (II) :
- Y représente le groupe suivant : 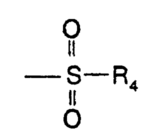 dans lequel R₄ représente un radical hydrocarboné ayant de 1 à 20 atomes de carbone et plus particulièrement : ,
   . un radical alkyle ayant de 1 à 10 atomes de carbone, de préférence, 1 à 4 atomes de carbone et plus préférentiellement un radical méthyle ou éthyle, éventuellement porteur d'un atome d'halogène, d'un groupe CF₃ ou d'un groupe ammonium N(R₅)₄, R₅, identiques ou différents, représentant un radical alkyle ayant de 1 à 4 atomes de carbone,
   . un radical cycloalkyle ayant de 3 à 8 atomes de carbone, de préférence, un radical cyclohexyle,
   . un radical aryle ayant de 6 à 12 atomes de carbone, de préférence, un radical phényle éventuellement porteur d'un radical alkyle ayant de 1 à 10 atomes de carbone, de préférence, 1 à 4 atomes de carbone et plus préférentiellement un radical méthyle ou éthyle, d'un atome d'halogène, d'un groupe CF₃ ou d'un groupe NO₂,
   . un groupe CX₃ dans lequel X représente un atome de fluor, de chlore ou de brome,
   . un groupe CF₂ - CF₃.
- A, R₁, R₂ et R₃, ont la signification donnée précédemment.

Un phénol protégé de formule (II) peut être obtenu par réaction, en présence d'une base, du composé phénolique de départ de formule (I) avec un agent protecteur répondant à la formule suivante (III) : 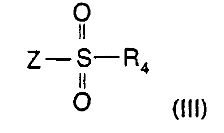 dans ladite formule (III) :
- R₄ a la signification donnée précédemment,
- Z représente :
   . un groupe hydroxyle ou un atome d'halogène, de préférence, un atome de chlore ou de brome,
   . un groupe -O-SO₂-R₄^{'}dans lequel R₄^{'}, identiques ou différents de R₄, a la signification donnée pour R₄.

Les agents protecteur préférés répondent à la formule (III) dans laquelle Z représente un atome de chlore ou de brome.

Les composés phénoliques mis en oeuvre préférentiellement sont plus particulièrement :
- la pyrocatéchine,
- la résorcine,
- l'o-crésol,
- le m-crésol,
- le 2-éthylphénol,
- le 3-éthylphénol,
- le 2-propylphénol
- le 2-sec-butylphénol,
- le 2-tert-butylphénol,
- le 3-tert-butylphénol,
- le 2-méthoxyphénol,
- le 3-méthoxyphénol,
- le 2-éthoxyphénol,
- le 3-éthoxyphénol,
- le 2-chlorophénol,
- le 3-chlorophénol,
- le 2,3-diméthylphénol,
- le 2,5-diméthylphénol,
- le 2,6-diméthylphénol,
- le 3,5-diméthylphénol,
- la vanilline,
- le 2,3-dichlorophénol,
- le 2,6-dichlorophénol,
- le 3,5-dichlorophénol,
- le pyrogallol,
- le 2,3,5-triméthylphénol,
- le 2,3,6-triméthylphénol,
- le 2,6-di-tert-butylphénol,
- le 3,5-di-tert butylphénol,
- le 2,3,6-trichlorophénol,
- le 1,2-dihydroxynaphtalène,
- le 1,4-dihydroxynaphtalène,
- le 1,5-dihydroxynaphtalène,
- le 2,3-dihydroxynaphtalène,
- le 2,6-dihydroxynaphtalène,
- le 2,7-dihydroxynaphtalène,
- le 4-méthoxy-1-naphtol,
- le 6-bromo-2-naphtol,
- le 2-phénoxyphénol,
- le 3-phénoxyphénol.

En ce qui concerne les agents protecteurs, on fait appel plus particulièrement aux composés suivants :
- l'anhydride triflique,
- le chlorure de méthanesulfonyle,
- le chlorure de trifluorométhanesulfonyle,
- le chlorure de benzènesulfonyle,
- le chlorure de p-toluènesulfonyle-

Intervient donc, dans le procédé de l'invention, une base qui peut être une base minérale ou organique dont le rôle est de pièger l'acidité.

On peut faire appel à n'importe quelle type de base.

La base a généralement un pKa supérieur à 7.

Le pKa est défini comme la constante de dissociation ionique du couple acide/base, lorsque l'eau est utilisée comme solvant.

Pour le choix d'une base ayant un pKa tel que défini par l'invention, on peut se reporter, entre autres, au HANDBOOK OF CHEMISTRY AND PHYSICS, 66^{ème} édition, p. D-161 ET D-162.

On peut faire appel à des bases fortes telles que les hydroxydes de métaux alcalins, de préférence, l'hydroxyde de sodium ou de potassium ou à des sels de métaux alcalins, notamment carbonates, bicarbonates, phosphates, hydrogénophosphates, sulfates, acétates et trifluoroacétates de métaux alcalins

La concentration de la solution basique de départ n'est pas critique. La solution d'hydroxyde de métal alcalin mise en oeuvre a une concentration généralement comprise entre 10 et 50 % en poids.

Dans le cas où l'agent protecteur est de type halogénure, une variante préférée de l'invention consiste à ajouter une base, de préférence, une amine tertiaire afin de piéger l'hydracide libéré.

Parmi les bases utilisables, il convient de citer les amines tertiaires et plus particulièrement celles répondant à la formule générale (IV)

N - (R₆)₃ (IV)

dans laquelle :
- les groupes R₆, identiques ou différents, représentent des restes hydrocarbonés ayant de 1 à 20 atomes de carbone, tels que des groupes alkyle, cycloalkyle, aryle ou hétérocyclique ;
- 2 groupes R₆ forment ensemble et avec l'atome d'azote un hétérocycle ayant 4 à 5 atomes.

Plus particulièrement :
- les symboles R₆ représentent un radical alkyle ayant 1 à 10 atomes de carbone et de préférence 1 à 4 atomes de carbone ou un radical cyclopentyle ou cyclohexyle ou un radical pyridinyle ;
- 2 groupes R₆ forment ensemble et avec l'atome d'azote un cycle pipéridine ou pyrrolidine.

A titre d'exemples de telles amines, on peut citer la triéthylamine, là tri-*n*-propylamine, la tri-n-butylamine, la méthyldibutylamine, la méthyldicyclohexylamine, l'éthyldiisopropylamine, la N,N-diéthylcyclohexylamine, la diméthylamino-4 pyridine, la N-méthylpipéridine, la N-éthylpipéridine, la N-n-butylpipéridine, la 1,2-diméthylpipéridine, la N-méthylpyrrolidine, la 1,2-diméthylpyrrolidine.

On conduit la réaction du composé phénolique, de l'agent protecteur, en présence d'une base, de préférence, dans un solvant organique.

On fait appel à n'importe quel type de solvant organique polaire ou apolaire ou à un mélange de solvants organiques.

On peut faire appel également à un solvant organique inerte dans les conditions de la réaction. Ainsi, on peut utiliser des hydrocarbures aliphatiques ou cycloaliphatiques, halogénés ou non tels que par exemple, l'hexane, le cyclohexane, le dichloroéthane ou aromatiques, halogénés ou non tels que le benzène, le toluène, les xylènes, les chlorobenzènes ; des esters tels que le benzoate de méthyle, le téréphtalate de méthyle, l'adipate de méthyle, le phtalate de dibutyle ; des esters ou éthers de polyols tels que le diacétate de tétraéthylèneglycol ; des éthers aliphatiques, linéaires ou cycliques tels que l'éther isopropylique, le tétrahydrofuranne ou le dioxanne ; des nitriles aliphatiques ou aromatiques comme l'acétonitrile, le propionitrile, le butanenitrile, l'isobutanenitrile, le benzonitrile, le cyanure de benzyle.

Pour ce qui est des différents réactifs et substrats, ils sont mis en oeuvre dans les quantités précisées ci-après.

La concentration du composé phénolique de formule (I) mis en oeuvre dans le solvant peut varier dans de très larges limites. Généralement, la concentration du composé phénolique varie entre 1 et 5 mol/l et est préférentiellement voisine de 3 mol/l de solvant.

La quantité de base mise en oeuvre, exprimée par le rapport du nombre d'équivalents de OH⁻ au nombre de moles de composé phénolique de formule (I) peut varier dans de larges limites. Ainsi, le rapport molaire OH-/composé phénolique peut varier entre 0,5 et 3,0, et de préférence entre 1,0 et 2,0.

La quantité d'agent protecteur mise en oeuvre exprimée par le rapport entre le nombre de moles d'agent protecteur et le nombre de moles de composé phénolique de départ varie avantageusement entre 1,0 et 1,5 et se situe de préférence, aux environs de 1,1.

La température à laquelle on réalise l'opération de protection du groupe hydroxyle du composé phénolique se situe entre 0°C et la température ambiante (le plus souvent entre 15°C et 25°C).

Selon un mode de réalisation pratique de l'invention, on peut effectuer le mélange de tous les réactifs dans un ordre quelconque.

Une réalisation préférentielle de l'invention consiste à ajouter la base dans un milieu réactionnel comprenant le composé phénolique, éventuellement un solvant organique et l'agent protecteur.

En fin de réaction, on obtient le composé phénolique sous forme protégée qui peut être purifié selon les méthodes classiques (extraction liquide-liquide, filtration).

### Réactions de substitution électrophile.

Conformément au procédé de l'invention, on effectue dans une deuxième étape la réaction de substitution électrophile par mise en contact du composé phénolique protégé avec un réactif électrophile.

Cette étape permet une fonctionnalisation par échange de l'atome d'hydrogène par un groupe fonctionnel comprenant un atome de carbone ou de soufre électrophile. Cet échange est effectué spécifiquement en position para du groupe électro-donneur.

L'échange entre l'atome d'hydrogène et le réactif électrophile peut se faire notamment dans le cas des réactions suivantes : les réactions d'amidoalkylation, les réactions de Friedel-Crafts telles que notamment acylation, sulfonylation, alkylation, les réactions d'aminoalkylation, d'hydroxyalkylation, de formylation, de carboxylation etc...

### Amidoalkylation.

On soumet le composé phénolique protégé à une réaction d'amidoalkylation. L'expression "amidoalkylation" doit être prise au sens large car l'on inclut également sous ce terme non seulement des groupes alkyle mais également des groupes arylalkyle.

Dans une étape suivante du composé de l'invention, on fait donc réagir le composé phénolique sous forme protégée, et répondant préférentiellement à la formule (II), en présence d'un acide fort, avec un amide ou un carbamate et un composé carbonylé répondant plus particulièrement à la formule générale N) : 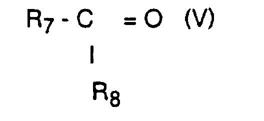 dans ladite formule (V) :
- R₇ représente un atome d'hydrogène,
- R₈ représente un atome d'hydrogène, un radical alkyle linéaire où ramifié ayant de 1 à 6 atomes de carbone, un radical cycloalkyle ayant de 3 à 8 atomes de carbone, un radical alcényle ayant de 2 à 6 atomes de carbone
ou un radical phényle éventuellement substitué ou un groupe électro-attracteur.

A titre d'exemples de groupes électro-attracteurs convenant à la présente invention, on peut citer :
- un groupe -CHO,
- un groupe acyle R₉-CO- ou R₉-CO-(CH₂)ₘ- dans lequel R₉ représente un radical alkyle linéaire ou ramifié ayant de 1 à 6 atomes de carbone et m représente un nombre de 1 à 3,
- un groupe -COOR₁₀ dans lequel R₁₀ représente un atome d'hydrogène ou un radical alkyle linéaire ou ramifié ayant de 1 à 6 atomes de carbone,
- un groupe -CX₂H dans lequel X représente un atome d'halogène, de préférence, un atome de fluor, chlore ou brome,
- un groupe -CX₃ dans lequel X représente un atome d'halogène, de préférence, un atome de fluor, chlore ou brome,
- un groupe nitro.

Comme exemples de composés carbonylés répondant à la formule (V), on peut citer :
- le formaldéhyde ou un générateur de formaldéhyde tel que, par exemple, le paraformaldéhyde pouvant provenir d'une solution aqueuse (méthylèneglycol) ou utilisé sous la forme de polyformaldéhydes linéaires de degré de polymérisation indifférent, ayant de préférence un nombre de motifs (CH₂O) compris entre 8 et 100 motifs,
- le glyoxal,
- l'acide glyoxylique,
- le bromal,
- le fluoral.

Parmi les composés carbonylés précités, le paraformaldéhyde est préféré.

On met en oeuvre ledit réactif soit sous forme solide ou soit sous forme d'une solution aqueuse ayant une concentration inférieure à 50 % en poids, de préférence, comprise entre 20 et 50 % en poids.

Conformément au procédé de l'invention, on fait réagir le composé phénolique protégé avec un composé carbonylé de formule (V), en présence d'un amide ou d'un carbamate.

Comme exemples d'amides ou de carbamates convenant à l'invention, on peut citer ceux qui répondent la formule (VI) suivante : 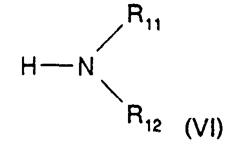 dans laquelle :
- au moins l'un des groupes R₁₁ et R₁₂, représente un groupe : 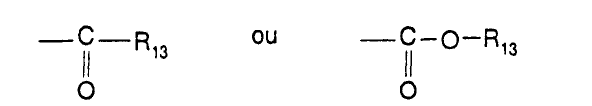
- les groupes R₁₁, R₁₂ et R₁₃, identiques ou différents, représentent :
   . un atome d'hydrogène,
   . un radical alkyle linéaire ou ramifié ayant de 1 à 12 atomes de carbone éventuellement porteur d'atomes d'halogène,
   . un radical cycloalkyle ayant de 3 à 8 atomes de carbone, de préférence, un radical cyclopentyle, un radical cyclohexyle, un radical cycloheptyle
   . un radical cycloalkylalkyle, de préférence, cyclopentylalkyle, cyclohexylalkyle, cycloheptylalkyle dont la partie alkyle comporte 1 à 4 atomes de carbone,
   . un radical alkylaryle, de préférence, un radical phénylalkyle, dont la partie alkyle comporte 1 à 4 atomes de carbone, de préférence, un radical benzyle,
- deux groupes R₁₁ et R₁₂ peuvent former avec les deux atomes qui les portent, un cycle carbocyclique, saturé, insaturé ou aromatique ayant de 3 à 8 atomes de carbone.

A titre d'exemples illustratifs, on peut citer, comme composés de formule (VI), l'acétamide, le chloroacétamide, le benzamide, le carbamate de benzyle.

Selon le procédé de l'invention, on conduit la réaction de l'amidoalkylation du composé phénolique protégé, en milieu acide.

On met en oeuvre selon l'invention, un acide protonique ayant un pKa inférieur ou égal à 4,00. Encore plus préférentiellement, on fait appel à un acide protonique ayant un pKa inférieur ou égal à 3,00.

Le pKa est défini comme la constante de dissociation ionique du couple acide/base, lorsque l'eau est utilisée comme solvant.

Pour le choix d'un acide ayant un pKa tel que défini par l'invention, on peut se reporter, entre autres, au HANDBOOK OF CHEMISTRY AND PHYSICS, 66^{ème} édition, p. D-161 ET D-162.

Comme exemples d'acides protoniques convenant à l'invention, on peut citer plus particulièrement les oxacides minéraux halogénés ou non, tels que l'acide sulfurique, l'acide chlorhydrique, l'acide chlorosulfonique, l'acide fluorosulfonique ; les acides phosphoriques tels que l'acide phosphorique, l'acide (2-éthylhexyl)phosphorique, l'acide (octylphényl)phosphorique ; les acides phosphoniques tels que, par exemple, l'acide (2-éthylhexyl)(2-éthylhexyl)phosphonique ; les acides carboxyliques perhalogénés ou non tels que l'acide formique, l'acide citrique, l'acide trichloroacétique, l'acide trifluoroacétique.

Les acides sulfoniques halogénés ou non sont également bien adaptés à la présente invention. Parmi ceux-ci, on peut citer l'acide fluorosulfonique, l'acide chlorosulfonique ou l'acide trifluorométhanesulfonique, l'acide méthanesulfonique, l'acide éthanesulfonique, l'acide éthanedisulfonique, l'acide benzènesulfonique, les acides benzènedisulfoniques, les acides toluènesulfoniques, les acides naphtalènesulfoniques, les acides naphtalènedisulfoniques, les acides camphorsulfoniques.

On utilise préférentiellement l'acide sulfurique.

La réaction est conduite comme l'étape précédente dans un solvant organique.

Les solvants organiques précités conviennent et mais l'on préfère faire appel à un acide aliphatique carboxylique.

L'acide carboxylique représente au moins 50 %, de préférence, de 60 à 100 % du poids de solvants organiques.

On peut mettre en oeuvre selon l'invention, tout acide monocarboxylique aliphatique saturé liquide dans les conditions réactionnelles, de préférence, liquide à la température ambiante. Par température ambiante, on entend généralement une température comprise entre 15 et 25 °C.

Afin de déterminer si un acide monocarboxylique aliphatique saturé convient à la présente invention, on peut se reporter à la littérature, notamment au HANDBOOK of CHEMISTRY and PHYSICS.

Comme exemples d'acides monocarboxylique aliphatiques saturés convenant bien à la présente invention, on peut citer, entre autres, l'acide acétique, l'acide propionique, l'acide butyrique, l'acide isobutyrique, l'acide pentanoïque, l'acide 2-méthylbutanoïque.

Parmi tous les acides monocarboxylique aliphatiques saturés, l'acide acétique est choisi préférentiellement.

En ce qui concerne les concentrations et les quantités de réactifs à mettre en oeuvre, on définit ci-après les conditions préférées.

Selon le procédé de l'invention, on fait réagir le composé carbonylé de formule (V) sur le composé phénolique protégé répondant préférentiellement à la formule (II), en présence d'un amide ou carbamate répondant préférentiellement à la formule (VI).

La concentration du composé phénolique protégé mis en oeuvre dans le solvant peut varier dans de très larges limites. Généralement, la concentration du composé phénolique varie entre 0,2 et 5,0 mol/l et est préférentiellement entre 0,2 et 2,0 mol/l de solvant.

On choisit préférentiellement une quantité de composé carbonylé en excès par rapport au composé phénolique protégé.

Le rapport entre le nombre de moles de composé carbonylé et le nombre de moles de composé phénolique protégé est d'au moins 1, de préférence, entre 1,0 et 2,0.

Pour ce qui est de la quantité d'amide ou de carbamate exprimée par rapport au composé carbonylé, elle est généralement en excès. Le rapport entre le nombre de moles d'amide ou de carbamate et le nombre de moles de composé carbonylé est choisi avantageusement entre 1,0 et 3,0 et de préférence, aux environs de 2,0.

La quantité d'acide fort mise en oeuvre est telle que le rapport molaire H⁺/composé phénolique protégé peut varier entre 1,0 et 20 et de préférence entre 2,0 et 3,0.

La réaction est conduite avantageusement entre la température ambiante et 120°C, de préférence, entre 40°C et 80°C.

On conduit généralement cette réaction d'amidoalkylation, sous la pression atmosphérique.

En fin de réaction, on obtient un produit amidoalkylé. Il se trouve généralement sous une forme pâteuse. On dilue le milieu avec un solvant quelconque (par exemple l'acétate d'éthyle), puis on ajoute une base, de préférence, la soude ou la potasse en une quantité telle que le pH se situe entre 7 et 10.

On extrait le produit amidoalkylé avec un solvant approprié, par exemple, acétate d'éthyle et/ou chloroforme.

On peut éventuellement purifier le produit obtenu par recristallisation dans un solvant approprié, par exemple, le chloroforme ou l'acétate d'éthyle.

A la fin de cette étape, on obtient un composé phénolique protégé amidoalkylé qui répond préférentiellement à la formule (VII) : 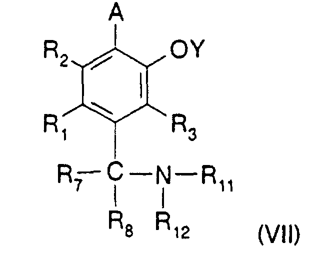 dans ladite formule, les différents symboles ont la signification donnée précédemment.

### Déprotection du groupe hydroxyle.

Conformément à l'invention, on effectue dans une dernière étape, le clivage du groupe sulfonyle, à l'aide d'une base afin de libérer le groupe hydroxyle.

Selon les conditions de déprotection, l'invention permet d'obtenir un composé phénolique porteur d'un groupe électro-donneur et d'un groupe amidoalkylé ou bien d'un groupe alkylamine, en position para par rapport au groupe électro-donneur.

A cet effet, on met en oeuvre une solution basique, de préférence, une solution aqueuse de soude, de potasse ou de carbonate de sodium ou de potassium.

La quantité de base exprimée par le rapport entre le nombre de moles de base et le nombre de moles de composé phénolique protégé et amidoalkylé est généralement d'au moins de 2, de préférence, entre 2 et 4.

On effectue cette opération de préférence dans un solvant organique. Les alcools, de préférence, les alcools aliphatiques et plus particulièrement le méthanol, l'éthanol et l'isopropanol sont des solvants de choix.

La concentration du composé phénolique protégé et amidoalkylé est avantageusement compris entre 0,5 et 1 mol/litre.

On chauffe généralement sous reflux, pendant une durée allant de 4 à 24 heures.

On élimine le solvant par distillation.

Après addition d'eau, le produit formé à savoir le composé phénolique amidoalkylé et déprotégé est extrait après acidification à pH de préférence, égal à 6, dans un solvant organique non miscible à l'eau, comme par exemple, le dichlorométhane ou l'acétate d'éthyle.

On récupère le produit après élimination du solvant réactionnel.

Selon une autre variante du procédé de l'invention, il est possible, non seulement au cours de cette opération de cliver sélectivement la fonction amide permettant ainsi d'obtenir une fonction alkylamine.

Le produit amidoalkylé et protégé obtenu à la fin de la deuxième étape et répondant préférentiellement à la formule (VII) est mis à réagir avec une solution d'acide minéral.

On peut faire appel notamment aux acides forts suivants : l'acide chlorhydrique, l'acide perchlorique, l'acide sulfurique, l'acide bromhydrique.

La quantité d'acide exprimée par le rapport entre le nombre de moles d'ions H⁺ et le nombre de moles de composé phénolique protégé et amidoalkylé varie généralement, entre 5 et 10.

La concentration de la solution d'acide n'est pas critique et l'on peut faire appel aussi bien à une solution d'acide diluée ou concentrée.

On chauffe comme précédemment mais de préférence, à la température de reflux.

On récupère un composé phénolique protégé porteur d'un groupe alkylamine en position para par rapport au groupe électro-donneur.

### Réactions de Friedel-Crafts : acylation, sulfonylation et alkylation.

Selon une variante du procédé de l'invention, on fait réagir le composé phénolique protégé selon les réactions de type Friedel-Crafts telles que notamment les réactions d'acylation, de sulfonylation ou d'alkylation.

Pour ce qui est du réactif d'acylation, on fait appel aux acides carboxyliques et leurs dérivés, halogénures ou anhydrides, de préférence, anhydrides.

En ce qui concerne l'agent de sulfonylation, on met en oeuvre plus particulièrement les halogénures ou les anhydrides de sulfonyle ou d'aminosulfonyle.

Les réactifs d'acylation ou de sulfonylation répondent plus particulièrement aux formules suivantes : 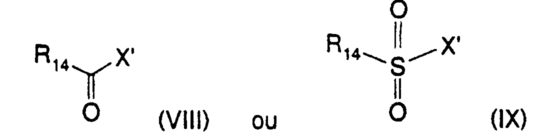
. dans les formules (VIII) ou (IX) :
   - R₁₄ représente un radical aliphatique saturé ou insaturé, linéaire ou ramifié ayant de 1 à 24 atomes de carbone ; un radical cycloaliphatique, saturé, insaturé ou aromatique, monocyclique ou polycyclique, ayant de 4 à 12 atomes de carbone ; un radical aliphatique saturé ou insaturé, linéaire ou ramifié, porteur d'un substituant cyclique,
      X' représente un atome d'halogène, de préférence un atome de chlore ou de brome,
. dans la formule (VIII) :
   - X' représente un radical -O-CO-R₁₅ avec R₁₅, identique ou différent de R₁₄, ayant la même signification que R₁₄,
. dans la formule (IX) :
   - X' représente un radical -O-SO₂-R₁₅ avec R₁₅, identique ou différent de R₁₄, ayant la même signification que R₁₄,
   - R₁₄ représente :

. un radical alkoxy R₁₆-O- avec R₁₆ ayant la même signification que R₁₄,
. un groupe amino (R₁₇)(R₁₈)-N- avec R₁₇, identique ou différent de R₁₈, ayant la même signification que R₁₄.

Par substituant cyclique, on entend de préférence, un cycle carbocyclique saturé, insaturé ou aromatique, de préférence cycloaliphatique ou aromatique notamment cycloaliphatique comprenant 6 atomes de carbone dans le cycle ou benzénique.

Plus préférentiellement, R₁₄ représente un radical alkyle linéaire ou ramifié ayant de 1 à 12 atomes de carbone, de préférence de 1 à 6 atomes de carbone : la chaîne hydrocarbonée pouvant être éventuellement interrompue par un hétéroatome (par exemple, l'oxygène), par un groupe fonctionnel (par exemple -CO-) et/ou porteuse d'un substituant (par exemple, un halogène ou un groupe CF₃).

R₁₄ représente de préférence un radical alkyle ayant de 1 à 4 atomes de carbone, tel que méthyle, éthyle, propyle, isopropyle, butyle, isobutyle, sec-butyle, tert-butyle.

Le radical R₁₄ représente également d'une manière préférentielle un radical phényle qui peut être éventuellement substitué. Il est nécessaire que ce radical soit plus désactivé que le composé phénolique car dans le cas contraire, on assisterait à l'acylation de l'agent d'acylation lui-même.

Comme exemples plus particuliers de substituants, on peut citer, notamment :
- un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
- un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy, isobutoxy, sec-butoxy, tert-butoxy,
- un groupe hydroxyle,
- un atome d'halogène, de préférence un atome de fluor, chlore ou brome.

Les agents d'acylation préférés répondent à la formule (VIII) dans laquelle X' représente un atome de chlore et R₁₄ représente un radical méthyle ou éthyle.

Lorsque l'agent d'acylation est un anhydride d'acide, les composés préférés répondent à la formule (VIII) dans laquelle R₁₄ et R₁₅ sont identiques et représentent un radical alkyle ayant de 1 à 4 atomes de carbone.

Pour ce qui est des agents de sulfonylation, ils répondent préférentiellement à la formule (IX) dans laquelle X' représente un atome de chlore ou un radical -O-SO₂-R₁₅ dans lequel R₁₅ représente un radical alkyle ayant de 1 à 4 atomes de carbone et R₁₄ représente un radical phényle ou naphtyle ou un radical R₁₆-O- ou (R₁₇)(R₁₈)-N- dans lequel R₁₆, R₁₇, et R₁₈ représentent un radical alkyle linéaire ou ramifié ayant de 1 à 4 atomes de carbone.

A titre illustratif d'agents d'acylation répondant à la formule (VIII), on peut citer plus particulièrement :
- le chlorure d'acétyle,
- le bromure d'acétyle,
- le chlorure de monochloroacétyle,
- le chlorure de dichloroacétyle,
- le chlorure de propanoyle,
- le chlorure d'isobutanoyle,
- le chlorure de pivaloyle,
- le chlorure de stéaroyle,
- le chlorure de crotonyle,
- le chlorure de benzoyle,
- les chlorures de chlorobenzoyle,
- le chlorure de p-nitrobenzoyle
- le chlorure d'o-nitrobenzoyle
- les chlorures de méthoxybenzoyle,
- les chlorures de naphtoyle,
- l'anhydride acétique,
- l'anhydride isobutyrique,
- l'anhydride trifluoroacétique,
- l'anhydride benzoïque.

Comme exemples d'agents de sulfonylation répondant à la formule générale (IX), on peut mentionner entre autres :
- le chlorure de benzènesulfonyle,
- le chlorure de p-chlorobenzènesulfonyle,
- le chlorure de fluorobenzènesulfonyle,
- le chlorure de nitrobenzènesulfonyle,
- le chlorure de méthoxybenzènesulfonyle,
- le chlorure de tosyle,
- le chlorure de diméthylsulfamoyle,
- le chlorure de méthoxysulfonyle,
- l'anhydride benzènesulfonique,
- l'anhydride p-toluènesulfonique.

En ce qui concerne l'agent d'alkylation, on met en oeuvre plus particulièrement un agent de type halogénure ou alcool

Dans le cas d'un agent d'alkylation de type halogénure, l'atome d'halogène peut être le chlore, le brome ou l'iode mais on choisit, de préférence, les deux premiers.

Il peut être représenter symboliquement par la formule générale (X) :

R₁₈ - X (X)

dans ladite formule (X) :
- X symbolise un groupe hydroxyle ou un atome d'halogène : lesdits groupe hydroxyle ou atome d'halogène ne devant pas être en position vinylique ou alcynyle,
- R₁₈, éventuellement substitué, représente un radical aliphatique acyclique, saturé ou insaturé, linéaire ou ramifié ; un radical cycloaliphatique, saturé ou insaturé, monocylique ou polycyclique ; un radical aliphatique saturé ou insaturé, linéaire ou ramifié, porteur d'un substituant cyclique.

Dans la formule (X), le radical R₁₈ représente plus particulièrement :
- un radical alkyle, alcényle, alcadiényle, alcynyle, linéaire ou ramifié ayant, de préférence, de 1 à 12 atomes de carbone ; la chaîne hydrocarbonée pouvant être éventuellement :
   . interrompue par l'un des groupes suivants :
      -O- , -CO- , -COO- , -OCOO- 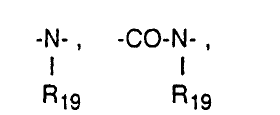 dans ces formules, R₁₉ représente un atome d'hydrogène ou un radical alkyle ayant de 1 à 4 atomes de carbone, un radical cyclohexyle ou phényle,
   . et/ou porteuse de l'un des substituants Y suivants :
      un groupe -OH, un groupe -COOH, un groupe -CHO, un groupe -NO₂, un groupe nitrile, un groupe amine -NH₂, un atome d'halogène de préférence, chlore ou brome, un groupe -CF₃.
- un radical cycloalkyle ou cycloalcényle ayant de préférence de 5 à 7 atomes de carbone éventuellement porteur d'un ou plusieurs substituants Y tels que précités et/ou d'un ou plusieurs substituants Z qui peuvent être : un radical alkyle linéaire ou ramifié ayant de 1 à 4 atomes de carbone, un radical alkoxy linéaire ou ramifié ayant de 1 à 4 atomes de carbone, un radical de formule :

   R₂₀-O-, R₂₀-COO-, R₂₀-O-CO-,

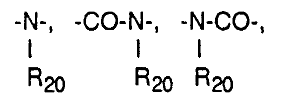

   dans lesdites formules, R₂₀ ayant la signification donnée précédemment; lesdits radicaux pouvant être polycycliques de type "pontés".
- un radical aliphatique acyclique, saturé ou insaturé, linéaire ou ramifié, tel que précisé ci-dessus°, porteur d'un substituant cyclique. Par cycle, on entend un cycle carbocyclique ou hétérocyclique, saturé, insaturé ou aromatique.

Le radical aliphatique peut être relié au cycle par un lien valentiel ou par l'un des groupes suivants : 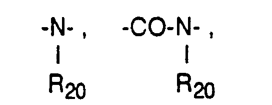 dans lesdites formules, R₂₀ ayant la signification donnée précédemment.

Comme exemples de cycles, on peut envisager :
- un radical cycloaliphatique saturé ou insaturé, monocyclique ou polycyclique tel que mentionné précédemment, de préférence, le radical cyclohexyle et le radical 1-cyclohexène-yle,
- un radical phényle, totale, xyxyle, éventuellement porteur d'un ou plusieurs substituants Y ou Z,
- un radical hétérocyclique monocyclique, saturé, insaturé ou aromatique et comportant en tant qu'hétéroatomes un ou plusieurs atomes d'oxygène, d'azote ou de soufre, contenant 4 à 6 atomes dans le cycle : ledit radical pouvant être porteur d'un ou plusieurs substituants Y ou Z,
- un radical constitué par un enchaînement de 2 à 4 groupes tels que définis précédemment liés entre eux par un lien valentiel et/ou par l'un des groupes suivants :
   -O-, -S-, 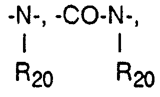 -CO-, -COO-, -SO₂-et/ou encore au moins un groupe de type alcoylène ou alcoylidène ayant de 1 à 4 atomes de carbone ; dans lesdites formules, R₂₀ représente un atome d'hydrogène, un radical alkyle ayant de 1 à 4 atomes de carbone, un radical cyclohexyle ou phényle,
- un radical aromatique constitué par un ensemble de 2 à 3 cycles aromatiques carbocycliques et/ou hétérocycliques et formant entre eux des systèmes orthocondensés, de préférence des noyaux benzéniques et pyridiniques.

Comme exemples de radicaux cyclo- ou arylaliphatiques R₁₈ porteurs d'un substituant cyclique, on peut citer préférentiellement les radicaux cyclo-hexylméthyle, cyclohexylbutyle, benzyle, 2-phényléthyle, 2-[4-(2-butyl)phényl]éthyle, styryle, α-phénylcyclohexylméthyle, phénoxyméthyle, phénoxyéthyle.

Dans la définition du radical R₁₈ du dérivé halogéné de formule (X), il est noté que celui-ci peut porter de un à plusieurs substituants Y ou Z. Par "plusieurs, on entend généralement un nombre total ne dépassant pas 3.

D'une manière préférentielle, le radical R₁₈ représente :
- un radical alkyle linéaire ou ramifié ayant de 1 à 8 atomes de carbone, tel que par exemple, le radical méthyle, éthyle, propyle, isopropyle, butyle, isobutyle, sec-butyle, tert-butyle, 2-éthylbutyle, pentyle, isopentyle, néopentyle, tert-pentyle, 2-méthylpentyle, hexyle, 2-éthylhexyle,
- un radical alcényle linéaire ou ramifié ayant de 2 à 8 atomes de carbone et comportant une double liaison éthylénique en β du groupe X comme par exemple, le radical allyle, 3-méthyl-2-butène-yle, 3-hexène-yle,
- un radical cyclohexyle éventuellement substitué par un radical alcoyle linéaire ou ramifié ayant de 1 à 4 atomes de carbone,
- un radical arylalkyle tel que symbolisé par la formule suivante 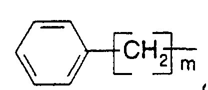 dans laquelle m est un nombre entier allant de 1 à 4, de préférence égal à 1.

Parmi les agents d'alkylation de type halogénure répondant à la formule (X), on peut citer tout particulièrement, les composés suivants :
- le bromure de méthyle
- le bromure d'éthyle
- le bromure de n-propyle
- le bromure d'isopropyle
- le bromobutane
- le bromohexane
- le bromoheptane
- le bromooctane
- le 1-bromo-2-méthylbutane
- le 1-bromo-3-méthylbutane
- le 2-bromo-2-méthylbutane
- le bromure d'allyle
- le chlorure d'allyle
- le chlorure de crotyle
- le 3-chloro-2-méthylpropène
- le 1-chloro-2-butène
- le 1-chloro-3-méthyl-2-butène
- le bromure de prényle
- le bromure de géranyle
- le chlorure de géranyle
- l'α-bromoacétone
- l'α,α-dibromoacétone
- l'α-chloroacétone
- l'α,α'-chloroacétone
- l'α-bromopropionate de méthyle
- l'α-bromopropionate de butyle
- l'α-chloropropionate de méthyle
- l'α-chloropropionate de butyle
- le bromure de cyclobutyle
- le bromure de cyclopentyle
- le bromure de cyclohexyle
- le bromure de cycloheptyle
- le (bromométhyl)cyclopropane
- le (2-chlorométhyl)pyridine
- le (3-chlorométhyl)pyridine
- le (4-chlorométhyl)pyridine
- le bromure de benzyle
- le chlorure de benzyle
- le (4-chlorométhyl)stilbène
- le (1-bromométhyl)naphtalène
- le (2-bromométhyl)naphtalène

Parmi les halogénures précités, les préférés sont les suivants :
- le bromure d'allyle
- le chlorure d'allyle
- le bromure de benzyle
- le chlorure de benzyle
- le bromure d'isopropyle
- le chlorure de crotyle
- le 1-chloro-2-butène
- le 3-chloro-2-méthylpropène
- le bromure de cyclohexyle.

En ce qui concerne le choix de l'halogénure utilisé, soit chlorure ou bromure, on le détermine en fonction de la nature de l'hydrocarbure dont dérive l'halogénure et d'impératifs économiques.

On peut faire appel, dans la mise en oeuvre du procédé de l'invention, à un composé bromé dans tous les cas.

Toutefois, les chlorures étant les moins chers, on cherchera à les utiliser préférentiellement, mais ceux-ci ne peuvent pas toujours être utilisés. En effet, il n'est pas possible selon l'invention d'avoir recours à un halogénure de type chlorure s'il y a présence d'une insaturation sur la chaîne hydrocarbonée portant l'atome de chlore, en position α par rapport à ce dernier : l'insaturation pouvant être une double ou une triple insaturation. Par exemple, le chlorure de benzyle peut être avantageusement utilisé.

Comme exemples d'agents d'alkylation de type alcool, on peut citer plus particulièrement :
- l'alcool benzylique
- l'alcool β-phényléthylique.

Les réactions d'acylation, sulfonylation ou d'alkylation sont conduites d'une manière classique, en présence d'un catalyseur de type Friedel-Crafts.

Le catalyseur intervenant dans le procédé de l'invention est un catalyseur de type Friedel-Crafts.

Une première classe de catalyseurs convenant à l'invention sont les acides de Lewis.

Comme exemples de sels organiques, on peut citer notamment l'acétate, le propionate, le benzoate, le méthanesulfonate, le trifluorométhanesulfonate des éléments métalliques ou métalloïdiques des groupes (IIIa), (IVa), (VIII), (IIb), (IIIb), (IVb), (Vb) et (VIb) de la classification périodique des éléments.

En ce qui concerne les sels inorganiques, on peut citer les chlorure, bromure, iodure, sulfate, oxyde et produits analogues des éléments métalliques ou métalloïdiques des groupes (IVa), (VIII), (IIb), (IIIb), (IVb), (Vb) et (Vlb) de la classification périodique des éléments.

Dans le présent texte, on se réfère ci-après à la Classification périodique des éléments publiée dans le Bulletin de la Société Chimique de France, n°1 (1966).

Les sels mis en oeuvre dans le procédé de l'invention sont plus particulièrement ceux des éléments du groupe (IIIa) de la classification périodique de préférence, le scandium, l'yttrium et les lanthanides ; du groupe (IVa) de préférence, le titane, le zirconium ; du groupe (VIII) de préférence, le fer ; du groupe (IIb) de préférence, le zinc ; du groupe (IIIb) de préférence, le bore, l'aluminium, le gallium, l'indium ; du groupe (IVb) de préférence, l'étain ; du groupe (Vb) de préférence, le bismuth ; du groupe (VIb) de préférence, le tellure.

Parmi les sels inorganiques, on peut citer les halogénures métalliques et préférentiellement le chlorure de zirconium, le chlorure ferrique, le chlorure de zinc, le chlorure d'aluminium, le bromure d'aluminium, le chlorure de gallium, le chlorure d'indium, le chlorure stannique, le chlorure de bismuth, le trifluorure de bore ; l'oxyde ferreux, l'oxyde ferrique, l'oxyde de gallium.

Il est possible de générer un halogénure in situ selon une méthode connue (PCT/FR98/00497 publié sous le n°98/40339).

Comme exemples préférés de catalyseurs, on peut citer le chlorure d'aluminium, le trifluorure de bore, le tétrachlorure de titane, le tétrachlorure d'étain.

En ce qui concerne les sels organiques, on met en oeuvre préférentiellement les sels de terres rares et/ou de bismuth de l'acide trifluorométhanesulfonique dénommé couramment "acide triflique".

Par "terre rare", on entend les lanthanides ayant un numéro atomique de 57 à 71 et l'yttrium ainsi que le scandium. '

Dans le procédé de l'invention, on envisage plus particulièrement les terres rares suivantes : le lanthane, l'ytterbium, le lutécium et/ou le scandium.

Les triflates de terres rares sont des produits connus qui sont décrits dans la littérature, notamment dans US-A-3 615 169. Ils sont généralement obtenus par réaction de l'oxyde de terre rare et de l'acide trifluorométhanesulfonique.

Les sels de bismuth de l'acide triflique décrits dans la demande de brevet WO 97/11930 (PCT/FR96/01488) peuvent être également mis en oeuvre dans le procédé de l'invention.

Une autre classe de catalyseurs convenant à l'invention sont les acides de Brônsted et l'on peut mentionner plus particulièrement l'acide sulfurique, l'acide fluorhydrique, l'acide chlorhydrique, les acides phosphoriques et les acides polyphosphoriques, les acides sulfoniques et notamment l'acide trifluorométhanesulfonique, l'acide perfluorosulfonique, l'acide fluorosulfonique.

Dans le procédé de l'invention, on peut mettre en oeuvre un catalyseur massique tel que précité ou sous forme supportée. A cet effet, le support peut être choisi parmi les oxydes de métaux, tels que les oxydes d'aluminium, de silicium et/ou de zirconium, les argiles et plus particulièrement, le kaolin, le talc ou la montmorillonite ou encore parmi les charbons éventuellement activés par un traitement bien connu à l'acide nitrique, le noir d'acétylène, ou les résines.

Le support peut être sous une forme quelconque, par exemple, poudre, billes, granulés, extrudés...

Dans le catalyseur, la teneur en phase active représente de 5 à 100 % du poids du catalyseur.

Conformément au procédé de l'invention, la réaction entre le composé phénolique et l'agent d'acylation, de sulfonylation ou d'alkylation, est conduite en phase liquide, en présence ou en l'absence d'un solvant organique : l'un des réactifs pouvant être utilisé comme solvant réactionnel.

Une variante préférée du procédé de l'invention consiste à conduire la réaction dans un solvant organique.

Plusieurs impératifs président au choix du solvant.

Il doit être inerte dans les conditions de l'invention, et présenter un point d'ébullition supérieur à la température de la réaction.

On fait appel de préférence, à un solvant organique, aprotique et peu polaire.

Comme exemples de solvants convenant à la présente invention, on peut citer en particulier les hydrocarbures aliphatiques ou aromatiques, halogénés ou non.

A titre d'exemples d'hydrocarbures aliphatiques, on peut citer plus particulièrement les paraffines tels que notamment, l'hexane, l'heptane ou le cyclohexane et les hydrocarbures aromatiques et plus particulièrement les hydrocarbures aromatiques comme notamment le benzène, le toluène, les xylènes, le cumène, les coupes pétrolières constituées de mélange d'alkylbenzènes notamment les coupes de type Solvesso®.

En ce qui concerne les hydrocarbures halogénés aliphatiques ou aromatiques, on peut mentionner plus particulièrement, les hydrocarbures perchlorés tels que notamment le tétrachlorométhane ; les hydrocarbures partiellement chlorés tels que le dichlorométhane, le chloroforme, le 1,2-dichloroéthane, le trichloroéthylène ; les hydrocarbures aromatiques halogénés tels que notamment le monochlorobenzène, les dichlorobenzènes et leurs mélanges.

On peut également utiliser un mélange de solvants organiques.

On effectue la réaction de substitution électrophile en mettant en jeu les réactifs dans les proportions mentionnées ci-après.

Le rapport entre le nombre de moles de composé phénolique et le nombre de moles d'agent d'acylation, de sulfonylation ou d'alkylation peut varier car le substrat peut servir de solvant réactionnel. Ainsi, le rapport peut aller de 0,1 à 10, de préférence aux environs de 4,0.

La quantité de catalyseur mis en oeuvre est déterminée de telle sorte que le rapport entre le nombre de moles de catalyseur et le nombre de moles d'agent d'acylation, de sulfonylation ou d'alkylation varie, de préférence, entre 0,001 et 1,0, et encore plus préférentiellement entre 0,02 et 0,2.

Pour ce qui est de la quantité de solvant organique mis en oeuvre, elle est choisie généralement de telle sorte que le rapport entre le nombre de moles de solvant organique et le nombre de moles de composé phénolique varie, de préférence, entre 0 et 100 et encore plus préférentiellement entre 0 et 50.

La température à laquelle est mise en oeuvre la réaction de Friedel-Crafts dépend de la réactivité du substrat de départ et de celle de l'agent d'acylation.

Elle se situe entre 20°C et 200°C, de préférence entre 40°C et 150°C.

Généralement, la réaction est conduite à pression atmosphérique mais des pressions plus faibles ou plus élevées peuvent également convenir.

D'un point de vue pratique, il n'y a pas de contraintes au niveau de la mise en oeuvre des réactifs. Ils peuvent être introduits dans un ordre quelconque.

Après mise en contact des réactifs, on porte le mélange réactionnel à la température souhaitée.

Une autre variante de l'invention consiste à chauffer l'un des réactifs (agent d'acylation, de sulfonylation ou d'alkylation ou composé phénolique) avec le catalyseur, puis à introduire l'autre réactif.

La durée de la réaction est fonction de nombreux paramètres. Le plus souvent, elle est de 30 minutes à 8 heures.

En fin de réaction, le milieu réactionnel est traité d'une manière classique pour éliminer le catalyseur, en particulier selon un traitement d'hydrolyse à l'eau.

On récupère le composé phénolique protégé acylé, sulfoné ou alkylé à partir de la phase organique selon les techniques connues, par élimination du solvant organique par distillation ou par cristallisation.

On peut conduire l'étape de déprotection du groupe hydroxyle, de préférence, en mettant en oeuvre le mode conduit en milieu basique qui est décrit ci-dessus.

### Aminoalkylation

Conformément au procédé de l'invention, on effectue la condensation d'un composé phénolique protégé de formule (II) avec un composé carbonylé de formule (XI), en présence d'une amine secondaire.

Intervient un composé carbonylé répondant à la formule générale (XI) : 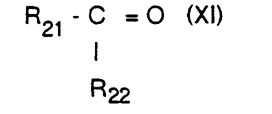 dans ladite formule (XI) :
- R₂₁ représente un atome d'hydrogène, un radical alkyle linéaire ou ramifié ayant de 1 à 6 atomes de carbone, un radical alcényle ayant de 2 à 6 atomes de carbone ou un radical phényle éventuellement substitué,
- R₂₂ peut être égal à R₂₁ ou représenter un groupe électro-attracteur.

A titre d'exemples de groupes électro-attracteurs convenant à la présente invention, on peut citer :
- un groupe -CHO,
- un groupe acyle R₂₃-CO- ou R₂₃-CO-(CH₂)ₘ- dans lequel R₂₃ représente un radical alkyle linéaire ou ramifié ayant de 1 à 6 atomes de carbone et m représente un nombre de 1 à 3,
- un groupe -COOR₂₄ dans lequel R₂₄ représente un atome d'hydrogène ou un radical alkyle linéaire ou ramifié ayant de 1 à 6 atomes de carbone,
- un groupe -CX₂H dans lequel X représente un atome d'halogène, de préférence, un atome de fluor, chlore ou brome,
- un groupe -CX₃ dans lequel X représente un atome d'halogène, de préférence, un atome de fluor, chlore ou brome.

Comme exemples de composés carbonylés répondant à la formule (XI), on peut citer :
- le formaldéhyde ou un générateur de formaldéhyde tel que, par exemple, le paraformaldéhyde provenant d'une solution aqueuse (méthylèneglycol) ou utilisé sous la forme de polyformaldéhydes linéaires de degré de polymérisation indifférent, ayant de préférence un nombre de motifs (CH₂O) compris entre 8 et 100 motifs,
- le glyoxal,
- l'éthanal,
- le propanal,
- le benzaldéhyde,
- l'acide glyoxylique,
- le chloral,
- le bromal,
- le fluoral,
- l'acétone,
- la méthyléthylcétone,
- la méthylisobutylcétone,
- la dichloroacétone,
- la trifluoroacétone,
- la trifluroacétophénone,
- la trifluoroacétylacétone,
- le pyruvate de méthyle,
- le pyruvate d'éthyle.

Parmi les composés carbonylés précités, le paraformaldéhyde est préféré.

On met en oeuvre ledit réactif généralement sous forme d'une solution aqueuse ayant une concentration inférieure à 50 % en poids, de préférence, comprise entre 20 et 50 % en poids. Il peut contenir quelques pourcentages d'un alcool, généralement le méthanol à une teneur inférieure à 15 % en poids.

Comme exemples d'amines convenant à l'invention, on peut citer celles qui répondent la formule (XII) suivante : 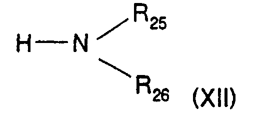 dans laquelle :
- R₂₅ et R₂₆, identiques ou différents, représentent :
   . un radical alkyle linéaire ayant de 1 à 12 atomes de carbone, un radical alkyle secondaire ayant 3 à 12 atomes de carbone ou un radical alkyle tertiaire ayant 4 à 12 atomes de carbone, ces radicaux alkyles pouvant comporter une ou deux fonctions éther - O - ou fonctions hydroxyle, amine tertiaire, acide carboxylique, ester d'acide carboxylique,
   . un radical phényle, un radical cyclohexyle, un radical cycloheptyle ou un radical cyclopentyle,
   . un radical phénylalkyle, cyclohexylalkyle, cycloheptylalkyle ou cyclopentylalkyle dont la partie alkyle comporte 1 à 4 atomes de carbone,
   . un atome d'hydrogène,
- R₂₅ ou R₂₆ forment ensemble et avec l'atome d'azote un hétérocycle, saturé ou comportant une ou plusieurs doubles liaisons, éventuellement substitué par un ou plusieurs radicaux alkyles ayant 1 à 4 atomes de carbone ;
- R₂₅ et R₂₆ forment ensemble, avec l'atome d'azote et avec un ou plusieurs atomes d'azote et/ou d'oxygène et/ou de soufre, un hétérocycle, saturé ou insaturé, éventuellement substitué par un ou plusieurs radicaux alkyles ayant 1 à 4 atomes de carbone ;
- R₂₅ et R₂₆ forment ensemble et avec l'atome d'azote un hétérocycle insaturé éventuellement substitué par un deux groupements méthyle ou éthyle ;
- R₂₅ et R₂₆ forment ensemble, avec l'atome d'azote et éventuellement avec un ou plusieurs atomes d'azote et/ou d'oxygène et/ou de soufre, un composé polycyclique, saturé ou insaturé, éventuellement substitué par un ou plusieurs radicaux alkyles ayant 1 à 4 atomes de carbone.

Parmi les amines de formule générale (XII), on préfère dans le cadre du présent procédé, les amines secondaires de formule (XIIa) : 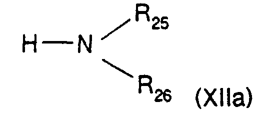 dans laquelle :
- R₂₅ et R₂₆, identiques ou différents, représentent :
   . un radical alkyle linéaire ayant 1 à 10 atomes de carbone ;
   . un radical alkyle secondaire ayant 3 à 10 atomes de carbone ;
   . un radical alkyle tertiaire ayant 4 à 10 atomes de carbone ;
   . un radical cyclohexyle ou cyclopentyle ;
   . un radical phényle ;
   . un radical benzyle ou phénéthyle ;
- R₂₅ et R₂₆ forment ensemble, avec l'atome d'azote et avec un autre atome d'azote et/ou d'oxygène, un hétérocycle saturé ou comportant une ou plusieurs insaturations ;
- R₂₅ et R₂₆ comportent une ou plusieurs fonctions amine, hydroxyle ou ester d'acide carboxylique.

A titre d'exemples illustratifs, on peut citer comme amines de formule (XII) ou (XIIa), la diméthylamine, la diéthylamine, la dipropylamine, la diisopropylamine, la dibutylamine, la diisobutylamine, la méthylpropylamine, la méthylbutylamine, la méthylisobutylamine, la méthyltertiobutylamine, la dilaurylamine, la méthylbenzylamine, la ditertiobutylamine, la 1-méthylcyclopentylamine, la 1-méthylcyclohexylamine, la dicyclohexylamine, la morpholine, l'imidazole, la pyrrolidine, l'imidazolidine, le pipérazine.

On choisit préférentiellement la diméthylamine, la diéthylamine, la dipropylamine, la diisopropylamine, la dibutylamine, la dilaurylamine.

En ce qui concerne les concentrations et les quantités de réactifs à mettre en oeuvre, on définit ci-après les conditions préférées.

Selon le procédé de l'invention, on fait réagir le composé carbonylé de formule (XI) sur le composé phénolique protégé de formule (II), en présence d'une amine secondaire.

On choisit préférentiellement une quantité de composé phénolique protégé en excès par rapport à l'amine secondaire.

Le rapport entre le nombre de moles de composé phénolique protégé de formule (II) et le nombre de moles d'amine secondaire de formule (XII) varie entre 0,9 et 3,0, de préférence, entre 1,0 et 2,0.

Pour ce qui est de la quantité d'amine secondaire, elle est généralement en excès par rapport au composé carbonylé. Le rapport entre le nombre de moles d'amine secondaire et le nombre de moles de composé carbonylé est choisi avantageusement entre 1,0 et 1,2.

Conformément au procédé de l'invention, on conduit la réaction, de préférence dans un solvant organique qui est de préférence, un solvant organique aprotique, peu polaire.

Comme exemples de solvants convenant à la présente invention, on peut citer en particulier les hydrocarbures aliphatiques ou aromatiques, halogénés ou non, les éther-oxydes aliphatiques, cycloaliphatiques ou aromatiques.

On peut utiliser également à titre de solvants organiques, les éther-oxydes aliphatiques, cycloaliphatiques ou aromatiques et, plus particulièrement, l'oxyde de diisopropyle, le méthyltertiobutyléther, le diméthyléther de l'éthylèneglycol (ou 1,2-diméthoxyéthane), le diméthyléther du diéthylèneglycol (ou 1,5-diméthoxy 3-oxapentane) ; l'oxyde de biphényle ou de benzyle ; le dioxane, le tétrahydrofuranne (THF).

Les solvants préférés sont : les hydrocarbures aromatiques, et plus spécialement le benzène et le toluène.

On peut également utiliser un mélange de solvants organiques.

La concentration du composés phénolique protégé dans le solvant peut varier dans de larges limites. Ainsi, on met généralement en oeuvre de 0,25 à 2 litres, de préférence, de 0,5 à 1 litre de solvant par mole de composé phénolique protégé.

Une variante du procédé de l'invention consiste à ajouter un catalyseur qui a pour rôle de faciliter la réaction entre l'amine secondaire et le composé carbonylé, et la réaction du produit résultant avec le composé phénolique protégé.

Comme exemples de catalyseurs susceptibles d'être mis en oeuvre, on fait appel entre autres, à l'acide sulfurique, aux acides sulfoniques halogénés ou pas et plus particulièrement aux acides halogénosulfoniques tels que l'acide fluorosulfonique, l'acide chlorosulfonique ou l'acide trifluorométhanesulfonique, l'acide méthanesulfonique, l'acide éthanesulfonique, l'acide éthanedisulfonique, l'acide camphorsulfonique, l'acide benzènesulfonique, les acides benzènedisulfoniques, les acides toluènesulfoniques, les acides naphtalènesulfoniques et les acides naphtalènedisulfoniques.

La quantité de catalyseur exprimée en mole de catalyseur par mole de composé phénolique protégé de formule (II) peut varier dans de larges limites. En général, une quantité de l'ordre de 1 % est mise en oeuvre.

La température à laquelle s'effectue la réaction est avantageusement choisie dans une gamme de température allant de 60°C à 120°C.

La réaction est conduite préférentiellement, sous pression atmosphérique.

Dans le cas où le solvant organique présente une température d'ébullition trop basse, il est possible de conduire le procédé de l'invention, sous pression.

Dans le cas inverse où le solvant possède une température d'ébullition trop élevée, on peut conduire le procédé de l'invention sous pression réduite avantageusement comprise entre 100 mm (13300 Pa) et 500 mm (46500 Pa) de mercure.

Selon une variante préférée du procédé de l'invention, on conduit le procédé de l'invention, sous atmosphère contrôlée de gaz inertes. On peut établir une atmosphère de gaz rares, de préférence l'argon mais il est plus économique de faire appel à l'azote.

D'un point de vue pratique, le procédé selon l'invention est simple à mettre en oeuvre.

Les différents réactifs peuvent être introduits dans n'importe quel ordre. D'une manière préférée, on choisit l'ordre des réactifs suivants : on introduit le composé carbonylé puis l'amine secondaire afin de former le réactif hémiaminal, puis le composé phénolique protégé et le catalyseur acide.

On porte le milieu réactionnel à la température désirée, tout en maintenant le milieu réactionnel sous agitation, à la température choisie dans l'intervalle précité pendant une durée variable allant de 1 à 10 heures.

Au cours de la réaction, il y a formation d'eau dans le milieu réactionnel. Une variante préférée de l'invention consiste à les éliminer du milieu réactionnel, au fur et à mesure de leur formation, par tout moyen connu, notamment par distillation azéotropique.

En fin de réaction, on obtient le composé phénolique protégé porteur d'un groupe aminoalkyle en position para par rapport au groupe électro-donneur

On peut conduire l'étape de déprotection du groupe hydroxyle, de préférence, en mettant en oeuvre le mode conduit en milieu basique qui est décrit ci-dessus.

### Hydroxyalkylation.

On peut dans une deuxième étape du procédé de l'invention mettre en oeuvre une réaction d'hydroxyalkylation.

A cet effet, on fait réagir le composé phénolique protégé répondant préférentiellement à la formule (II) avec un composé carbonylé, en présence d'un catalyseur adéquate.

Le composé carbonylé suceptible d'être mis en oeuvre répond plus particulièrement à la formule générale (XI) précisée ci-dessus.

Des exemples plus précis de composés carbonylés sont également donnés.

Parmi les composés carbonylés précités, le formaldéhyde est préféré.

On met en oeuvre ledit réactif généralement sous forme d'une solution aqueuse ayant une concentration inférieure à 50 % en poids, de préférence, comprise entre 20 et 50 % en poids.

On effectue la réaction d'hydroxyalkylation en présence d'un catalyseur classiquement utilisé pour une telle réaction.

On peut faire appel à un catalyse homogène en utilisant notamment l'un des acides de Brônsted précédemment cité.

Il est également possible de mettre en oeuvre un catalyseur hétérogène et l'on peut citer plus particulièrement les zéolithes et en particulier celles qui sont décrites dans la littérature et notamment dans EP-A-0 773 918.

Parmi toutes ces zéolithes, on fait appel préférentiellement dans le procédé de l'invention aux zéolithes β et aux mordénites.

Les zéolithes mises en oeuvre dans le procédé de l'invention, sont des produits connus décrits dans la littérature [cf. Atlas of zeolites structure types by W. M. Meier and D. H. Olson published by the Structure Commission of the International Zeolite Association (1992)].

Il peut être nécessaire d'effectuer un traitement classique de désalumination de la zéolithe ou bien lui faire subir un traitement afin de la rendre acide en particulier à l'aide d'un acide tel que notamment l'acide chlorhydrique, l'acide sulfurique, l'acide nitrique, l'acide perchlorique, l'acide phosphorique et l'acide trifluorométhanesulfonique.

La zéolithe constitue la phase catalytique. Elle peut être utilisée seule ou en mélange avec une matrice minérale. A cet effet, la matrice peut être choisie parmi les oxydes de métaux, tels que les oxydes d'aluminium, de silicium et/ou de zirconium, ou encore parmi les argiles et plus particulièrement, le kaolin, le talc ou la montmorillonite.

Dans le catalyseur, la teneur en phase active représente de 5 à 100 % du poids du catalyseur.

Les catalyseurs peuvent se présenter sous différentes formes dans le procédé de l'invention : poudre, produits mis en forme tels que granulés (par exemple, extrudés ou billes), pastilles, qui sont obtenus par extrusion, moulage, compactage ou tout autre type de procédé connu.

La réaction du composé phénolique protégé de formule (II) avec le composé carbonylé de formule (XI) est conduite, de préférence, en milieu aqueux lorsque l'on fait appel au formaldéhyde. Toutefois, il est également possible de faire la réaction au sein d'un liquide organique inerte dans les conditions réactionnelles choisies.

Comme exemples de solvants convenant à la présente invention, on peut citer en particulier les hydrocarbures aliphatiques ou aromatiques, halogénés ou non, les éther-oxydes aliphatiques, cycloaliphatiques ou aromatiques.

On peut faire appel à des solvants aprotiques polaires tels que les composés nitrés comme par exemple, le nitrobenzène ; les nitriles aliphatiques ou aromatiques comme l'acétonitrile ; la tétraméthylènesulfone (sulfolane).

On peut également utiliser un mélange de solvants organiques.

La concentration du composé phénolique protégé dans le milieu peut varier dans de larges limites. Ainsi elle peut être comprise entre 0,1 et 5'moles par litre de milieu et, de préférence, entre 0,2 et 2 moles par litre.

Le catalyseur peut représenter en poids par rapport au composé phénolique protégé engagé, de 5 à 80 %, de préférence, de 10 à 50 %.

La quantité de composé carbonylé de formule (XI) exprimée en moles de composé carbonylé par mole de composé phénolique protégé de formule (II) peut, elle aussi, varier dans de larges limites. Le rapport molaire composé carbonylé de formule (XI)/composé phénolique protégé de formule (II) peut varier entre 1 et 50. La borne supérieure ne présente aucun caractère critique mais toutefois pour des raisons économiques, il n'y a aucun intérêt à la dépasser.

La température de la réaction d'hydroxyalkylation est choisie, avantageusement entre 20°C et 100°C et encore plus préférentiellement entre 40°C et 90°C.

Généralement, la réaction est conduite à pression atmosphérique mais des pressions plus élevées peuvent également convenir allant de 1 à 50 bar, de préférence, de 1 à 25 bar. On travaille sous pression autogène lorsque la température de réaction est supérieure à la température d'ébullition des réactifs et/ou des produits.

On préfère conduire la réaction sous atmosphère contrôlée de gaz inertes tels que l'azote ou les gaz rares, par exemple l'argon.

La durée de la réaction peut être très variable. Elle se situe, le plus souvent, entre 15 minutes et 10 heures, de préférence entre 30 minutes et 5 heures.

D'un point de vue pratique, le procédé peut être mis en oeuvre en discontinu ou en continu.

On peut charger le catalyseur, le composé carbonylé de formule (XI), éventuellement un solvant organique puis l'on introduit le composé phénolique protégé. Un mode préféré de l'invention, consiste à introduire progressivement le composé phénolique protégé, en continu ou par fractions puis l'on porte le mélange réactionnel à la température souhaitée.

En fin de réaction, on récupère une phase liquide comprenant le composé phénolique protégé hydroxyalkylé en para du groupe électro-donneur qui peut être récupéré de manière classique.

Selon une variante de l'invention, il est possible d'oxyder le composé phénolique protégé porteur d'un groupe hydroxyalkylé en groupe formyle selon un procédé qui consiste à l'oxyder, en phase liquide, à l'aide d'oxygène moléculaire ou un gaz en contenant, en présence d'un catalyseur à base d'un métal M choisi parmi les métaux du groupe 8 de la classification périodique comprenant éventuellement, à titre d'activateurs des métaux tels que le cadmium, le cérium, le bismuth, le plomb, l'argent, le tellure ou l'étain.

On peut se référer à ce qui est décrit dans la littérature et notamment dans EP-A-0 773 918.

On peut conduire l'étape de déprotection du groupe hydroxyle, de préférence, en mettant en oeuvre le mode conduit en milieu basique qui est décrit ci-dessus.

### Formylation.

Une autre réaction consiste à faire réagir le composé phénolique protégé répondant préférentiellement à la formule (II) avec notamment le chloral ou l'hexaméthylènetétramine.

On met en oeuvre lesdits réactifs en une quantité exprimée par rapport au composé phénolique au moins égale à la quantité stoechiométrique et le plus souvent en excès pouvant atteindre par exemple 200 %.

La réaction peut être conduite en masse dans un acide tel que par exemple l'acide trifluroroacétique, l'acide sulfurique, l'acide fluorhydrique, l'acide borique.

Généralement, on on met en oeuvre une quantité d'acide égale à deux fois la quantité stoechiométrique exprimée par rapport au composé phénolique protégée.

La réaction est conduite avantageusement entre 60 et 100°C.

On récupère le composé phénolique protégé avec un groupe formyle en para du groupe électro-donneur.

On peut conduire l'étape de déprotection du groupe hydroxyle, de préférence, en mettant en oeuvre le mode conduit en milieu basique qui est décrit ci-dessus.

L'invention s'applique à toutes les réactions de substitution électrophile permettant d'introduire une liaison C-C ou C-S.

Elle n'est pas limitée aux réactions précédemment mentionnées qui ne constituent que des exemples de réaction de substitution électrophile.

On donne ci-après des exemples de réalisation du procédé de l'invention.

### Exemple 1

On réalise la préparation du composé (A) suivant :

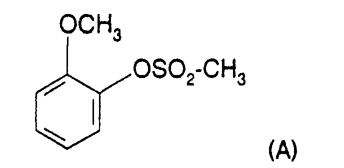

Dans un bicol de 1 litre agité par un agitateur magnétique et équipé d'une ampoule de coulée, on charge 50 g de gaïacol, 100 ml d'éther.

On ajoute à l'aide d'une seringue, 46,14 g de chlorure de méthanesulfonyle et l'on agite.

On additionne sur 3 heures, goutte à goutte, 56 ml de triéthylamine diluée dans 20 ml d'éther.

On filtre les sels de triéthylamine qui ont précipités.

On récupère les eaux-mères que l'on concentre.

On note la précipitation du gaïacol mésylé.

On effectue deux recristallisations dans l'éther et l'on cristallise à froid (-20°C).

On récupère 76,27 g d'un produit blanc ayant un point de fusion de 35-37°C

### Exemple 2

On réalise la préparation du composé (B) suivant :

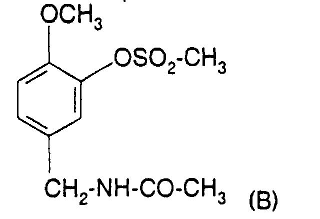

Dans un réacteur, on charge :
- 8,19 g d'acétamide,
- 2,2 g de paraformaldéhyde,
- 9 ml d'acide acétique,
- 6 ml d'acide sulfurique.

Quand la solution est limpide, c'est-à-dire que le paraformaldéhyde est tout dissout, on ajoute le gaïacol mésylé (2-méthylsulfone-1-méthoxyphénol).

On chauffe pendant 15 heures à la température du bain d'huile qui est de 80-90°C.

On laisse le milieu revenir à la température ambiante et l'on dilue avec de l'acétate d'éthyle (50 ml).

On ajoute une solution aqueuse de potasse 2 N jusqu'à ce que le pH soit égal à environ 8.

On extrait le produit formé successivement deux fois (2 x 50 ml) avec de l'acétate d'éthyle et 1 fois (50 ml) avec du trichlorométhane.

On réunit les fractions organiques.

On les sèche sur sulfate de magnésium.

On concentre le produit obtenu à l'évaporateur rotatif.

On cristallise le produit dans l'acétate d'éthyle (ou le trichlorométhane).

On obtient le produit (B) avec un rendement exprimé par rapport au gaïacol mésilyé de 80 %.

### Exemple 3

Dans cet exemple, on prépare le composé (C) suivant par déprotection du composé (B) obtenu dans l'exemple 2.

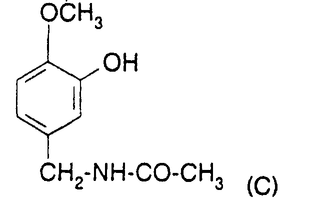

On dissout 4 g de produit de départ (B) dans 20 ml d'isopropanol dégazé.

On y ajoute alors 40 ml d'une solution aqueuse de potasse à 5 % en poids, également dégazée.

On chauffe à reflux pendant 6 heures, la température du bain étant de 100-110°C.

On concentre le mélange réactionnel brut obtenu à l'évaporateur rotatif.

On lave le solide obtenu à l'aide de 50 ml de trichlorométhane.

On ajoute 5 g de carbonate de potassium et on laisse encore tourner environ 1 heure.

On filtre le produit brut obtenu sur papier filtre.

On concentre le filtrat à l'évaporateur rotatif et on élimine les traces de solvant sous pression réduite (10 mm de mercure).

On récupère alors un solide blanc jaune (C) à raison de 2,5713 g, ce qui correspond à un rendement de 90 %.

On confirme la structure du produit (C) obtenu par RMN dans CDCl₃.

### Exemple 4

On prépare le composé (D) par traitement acide du composé (B).

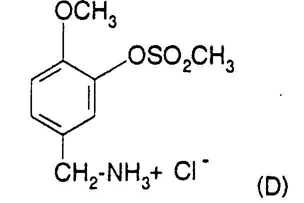

On prépare une solution aqueuse d'acide chlorhydrique 2 N que l'on dégaze préalablement.

On dissout le produit de départ dans 50 ml de cette solution d'acide chlorhydrique.

On chauffe pendant 6 heures, à la température du bain d'huile (110°C).

On concentre le mélange réactionnel brut à l'évaporateur rotatif.

On confirme la structure par RMN dans CDCl₃.

### Exemple 5

On conduit la réaction de formylation suivante : 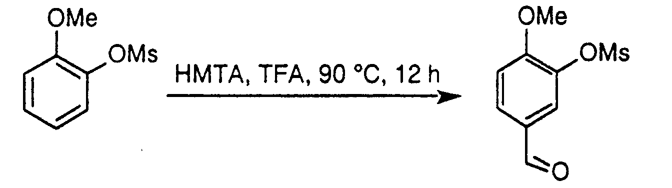 (Ms = OSO₂CH₃)

Dans un ballon on introduit successivement le gaïacol mésylé (0,5 g, 2.5 mmol), de l'hexaméthylènetétramine (0,35 g, 2.5 mmol) et de l'acide trifluoroacétique (3 ml).

Le mélange est chauffé à reflux pendant 12 heures, puis ramené à température ambiante.

20 ml d'eau glacée sont alors ajoutés.

Après 15 min d'agitation, le pH est ramené entre 8 et 9 par ajout d'une solution saturée de carbonate de sodium.

La phase aqueuse est alors extraite à l'éther (3 x 50 ml).

Les phases organiques sont rassemblées, séchées sur sulfate de sodium puis concentrées sous pression réduite (10 mm de mercure).

Le produit est obtenu avec un rendement de 90 % après purification par chromatographie sur gel de silice en utilisant comme éluant un mélange acétate d'éthyle / hexane 5/5. RMN ¹H (300 MHz, CDCl₃) δ (ppm) : 3.25 (3H, s), 4.00 (3H, s), 7.17 (1H, d, J = 8.3 Hz), 7.87 (2H, d, 8.3 Hz), 9.8 (1 H, s)

### Exemple 6

On conduit la réaction d'acylation de Friedel-Crafts suivante :

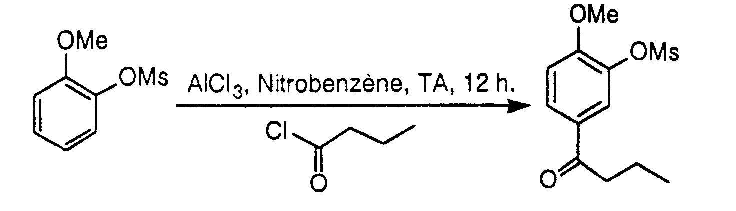

Un mélange de chlorure de butyryle (0,42 g, 4 mmol) et de gaïacol mésylé (0,8 g, 4 mmol) est additionné goutte à goutte à - 5 °C en 40 min sur du chlorure d'aluminium (0,64 g, 4,8 mmol) en solution dans du nitrobenzène (6 ml).

On laisse revenir la température à l'ambiante (TA) et le mélange réactionnel est maintenu sous agitation pendant 12 heures.

Après ce temps, 25 ml d'eau sont additionnés puis le pH est ramené entre 8 et 9 à l'aide d'une solution saturée de carbonate de sodium.

La phase aqueuse est extraite trois fois à l'acétate d'éthyle (50 ml).

Les phases organiques sont rassemblées, séchées sur sulfate de sodium puis concentrées sous pression réduite (10 mm de mercure).

Le produit est obtenu avec un rendement de 65 % après purification par chromatographie sur gel de silice en utilisant comme éluant un mélange acétate d'éthyle / hexane 8/2.
RMN ¹H (300 MHz, CDCl₃) δ (ppm) : 1.04 (3H, t, J = 7.3 Hz), 1.78 (2H, m), 2.9 (2H, t, J = 7.3 Hz), 3.24 (3H, s), 3.98 (3H, s), 7.08 (1 H, d, J = 8.3 Hz), 7.93 (2H, m).

## Revendications

1. Procédé de fonctionnalisation en position para par rapport à un groupe électro-donneur porté par un composé phénolique qui comprend une première étape de protection du groupe hydroxyle, une deuxième étape de réaction du composé phénolique protégé avec un réactif électrophile, éventuellement une troisième étape de déprotection du groupe hydroxyle **caractérisé par le fait que** l'on effectue la protection du groupe hydroxyle d'un composé phénolique porteur d'un groupe électro-donneur sous la forme d'une fonction ester sulfonique.

2. Procédé selon la revendication 1 **caractérisé par le fait que** le composé phénolique de départ est porteur d'au moins un groupe électro-donneur en position ortho par rapport au groupe hydroxyle et d'un atome d'hydrogène en position para du groupe électro-donneur.

3. Procédé selon l'une des revendications 1 et 2 **caractérisé par le fait que** le composé phénolique de départ répond à la formule générale (I) : 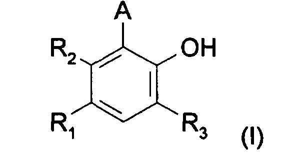 dans ladite formule (I) :
- A représente un groupe électro-donneur,
- R₁, R₂ et R₃, identiques ou différents, représentent :
. un atome d'hydrogène,
. un radical alkyle linéaire ou ramifié ayant de 1 à 20 atomes de carbone éventuellement porteur de un ou plusieurs atomes d'halogène,
. un groupe cycloalkyle ayant de 3 à 8 atomes de carbone, et de préférence, 6 atome de carbone,
. un radical aralkyle ayant de 6 à 20 atomes de carbone,
. un radical aryle ayant de 6 à 20 atomes de carbone,
. un atome d'halogène,
. un groupe électro-attracteur,
- deux groupes R₁ et R₂ peuvent former avec les deux atomes qui les portent, un cycle carbocyclique, saturé, insaturé ou aromatique ayant de 3 à 8 atomes de carbone.

4. Procédé selon la revendication 3 **caractérisé par le fait que** le groupe électro-attracteur est un groupe carboxylique ou ester (ayant de préférence, de 3 à 8 atomes de carbone) ou un groupe nitrile ou un groupe nitro.

5. Procédé selon l'une des revendications 1 à 4 **caractérisé par le fait que** le composé phénolique de départ répond à la formule (I) dans laquelle R₁, R₂ et R₃ représentent un atome d'hydrogène, un groupe alkyle linéaire ou ramifié ayant de 1 à 4 atomes de carbone, un atome d'halogène ou un groupe trifluorométhyle.

6. Procédé selon l'une des revendications 1 à 5 **caractérisé par le fait que** le composé phénolique de départ répond à la formule (I) dans laquelle A représente l'un des groupes ou fonctions suivantes :
- un groupe alkyle linéaire ou ramifié ayant de préférence de 1 à 6 atomes de carbone et encore plus préférentiellement de 1 à 4 atomes de carbone,
- un groupe cycloalkyle ayant de 3 à 8 atomes de carbone, et de préférence, 6 atome de carbone,
- le radical phényle,
- le radical benzyle ou phényléthyle,
- le groupe hydroxyle,
- un atome de fluor,
- un groupe alkoxy ayant de 1 à 6 atomes de carbone encore plus préférentiellement de 1 à 4 atomes de carbone dans la partie alkyle ou un groupe phénoxy,
- un groupe amino de préférence disubstitué dans lesquels les substituants identiques ou différents sont des radicaux alkyle linéaire ou ramifié ayant de 1 à 6 atomes de carbone et encore plus préférentiellement de 1 à 4 atomes de carbone ou phényle,
- un groupe alkylamide ou arylamide dans lequel le groupe alkyle a de 1 à 6 atomes de carbone et de préférence de 1 à 4 atomes de carbone et le groupe aryle a de 6 à 12 atomes de carbone et est de préférence, un groupe phényle.

7. Procédé selon l'une des revendications 1 à 6 **caractérisé par le fait que** le composé phénolique de départ répond à la formule (I) dans laquelle A représente un groupe méthoxy ou éthoxy.

8. Procédé selon l'une des revendications 1 à 7 **caractérisé par le fait que** le composé phénolique de départ est choisi parmi :
. la pyrocatéchine,
. la résorcine,
. l'o-crésol,
. le m-crésol,
. le 2-éthylphénol,
. le 3-éthylphénol,
. le 2-propylphénol
. le 2-sec-butylphénol,
. le 2-tert-butylphénol,
. le 3-tert-butylphénol,
. le 2-méthoxyphénol,
. le 3-méthoxyphénol,
. le 2-éthoxyphénol,
. le 3-éthoxyphénol,
. le 2-chlorophénol,
. le 3-chlorophénol,
. le 2,3-diméthylphénol,
. le 2,5-diméthylphénol,
. le 2,6-diméthylphénol,
. le 3,5-diméthylphénol,
. la vanilline,
. le 2,3-dichlorophénol,
. le 2,6-dichlorophénol,
. le 3,5-dichlorophénol,
. le pyrogallol,
. le 2,3,5-triméthylphénol,
. le 2,3,6-triméthylphénol,
. le 2,6-di-tert-butylphénol,
. le 3,5-di-tert butylphénol,
. le 2,3,6-trichlorophénol,
. le 1,2-dihydroxynaphtalène,
. le 1,4-dihydroxynaphtalène,
. le 1,5-dihydroxynaphtalène,
. le 2,3-dihydroxynaphtalène,
. le 2,6-dihydroxynaphtalène,
. le 2,7-dihydroxynaphtalène,
. le 4-méthoxy-1-naphtol,
. le 6-bromo-2-naphtol,
. le 2-phénoxyphénol,
. le 3-phénoxyphénol.

9. Procédé selon la revendication 1 **caractérisé par le fait que** dans une première étape, on protége la fonction hydroxyle en la transformant en ester sulfonique.

10. Procédé selon la revendication 9 **caractérisé par le fait que** les groupes ester sulfonique préférés sont :
- les tosylates (p-toluènesulfonates) -OSO₂-C₆H₄-CH₃
- les brosylates (p-bromobenzènesulfonates) -OSO₂-C₆H₄-Br
- les nosylates (p-nitrobenzènesulfonates) -OSO₂-C₆H₄-NO₂
- les mésylates (méthanesulfonates) -OSO₂-CH₃
- les bétylates (ammonioalcanesulfonates) -OSO₂-(CH₂)ₙNMe₃⁺ avec n compris entre 0 et 6,
- les triflates (trifluorométhanesulfonates) -OSO₂-CF₃
- les nonaflates (nonafluorobutanesulfonates) -OSO₂-C₄F₉
- les trésylates (2,2,2-trifluroéthanesulfonates).-OSO₂-CH₂-CF₃

11. Procédé selon l'une des revendications 1 à 10 **caractérisé par le fait que** le composé phénolique, sous forme protégée répond à la formule générale (II) : 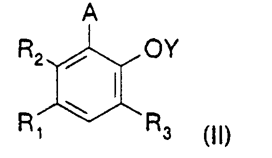 dans ladite formule (II) :
- Y représente le groupe suivant : 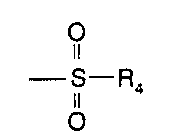 dans lequel R₄ représente un radical hydrocarboné ayant de 1 à 20 atomes de carbone et plus particulièrement :
. un radical alkyle ayant de 1 à 10 atomes de carbone, de préférence, 1 à 4 atomes de carbone et plus préférentiellement un radical méthyle ou éthyle, éventuellement porteur d'un atome d'halogène, d'un groupe CF₃ ou d'un groupe ammonium N(R₅)₄, R₅, identiques ou différents, représentant un radical alkyle ayant de 1 à 4 atomes de carbone,
. un radical cycloalkyle ayant de 3 à 8 atomes de carbone, de préférence, un radical cyclohexyle,
. un radical aryle ayant de 6 à 12 atomes de carbone, de préférence, un radical phényle éventuellement porteur d'un radical alkyle ayant de 1 à 10 atomes de carbone, de préférence, 1 à 4 atomes de carbone et plus préférentiellement un radical méthyle ou éthyle, d'un atome d'halogène, d'un groupe CF₃ ou d'un groupe NO₂,
. un groupe CX₃ dans lequel X représente un atome de fluor, de chlore ou de brome,
. un groupe CF₂ - CF₃.
- A, R₁, R₂ et R₃, ont la signification donnée précédemment.

12. Procédé selon la revendication 11 **caractérisé par le fait que** le phénol protégé de formule (II) est obtenu par réaction, en présence d'une base, du composé phénolique de départ de formule (I) avec un agent protecteur répondant à la formule suivante (III) : 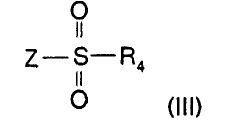 dans ladite formule (III) :
- R₄ a la signification donnée précédemment,
- Z représente :
. un groupe hydroxyle ou un atome d'halogène, de préférence, un atome de chlore ou de brome,
. un groupe -O-SO₂-R₄^{'} · dans lequel R₄^{'}, identiques ou différents de R₄, a la signification donnée pour R₄.

13. Procédé selon la revendication 12 **caractérisé par le fait que** l'agent protecteur répond à la formule (III) dans laquelle Z représente un atome de chlore ou de brome.

14. Procédé selon l'une des revendications 12 et 13 **caractérisé par le fait que** l'agent protecteur est choisi parmi :
. l'anhydride triflique,
. le chlorure de méthanesulfonyle,
. le chlorure de benzènesulfonyle,
. le chlorure de p-toluènesulfonyle.

15. Procédé selon l'une des revendications 9 à 14 **caractérisé par le fait que** l'on met en oeuvre une base minérale ou organique.

16. Procédé selon la revendication 15 **caractérisé par le fait que** la base mise en oeuvre est un hydroxyde de métal alcalin de préférence, l'hydroxyde de sodium ou de potassium ; un carbonate, un bicarbonate, un phosphate ou un hydrogénophosphate, un acétate, un trifluoroacétate de métal alcalin, de préférence, de sodium ou de potassium : une amine tertiaire.

17. Procédé selon la revendication 16 **caractérisé par le fait que** l'amine tertiaire répond à la formule générale (IV) :
N - (R₆)₃ (IV)
dans laquelle :
- les groupes R₆, identiques ou différents, représentent des restes hydrocarbonés ayant de 1 à 20 atomes de carbone, tels que des groupes alkyle, cycloalkyle, aryle ou hétérocyclique ;
- 2 groupes R₆ forment ensemble et avec l'atome d'azote un hétérocycle ayant 4 à 5 atomes.

18. Procédé selon la revendication 17 **caractérisé par le fait que** l'amine est la triéthylamine, la tri-n-propylamine, la tri-n-butylamine, la méthyldibutylamine, la méthyldicyclohexylamine, l'éthyldiisopropylamine, la N,N-diéthylcyclohexylamine, la diméthylamino-4 pyridine, la N-méthylpipéridine, la N-éthylpipéridine, la N-n-butylpipéridine, la 1,2-diméthylpipéridine, la N-méthylpyrrolidine, la 1,2-diméthylpyrrolidine.

19. Procédé selon l'une des revendications 9 à 18 **caractérisé par le fait que** l'on conduit la réaction de protection du composé phénolique, dans un solvant organique, polaire ou apolaire, de préférence, un hydrocarbure aliphatique, cycloaliphatique, halogéné ou non ; un ester ; un éther ou nitrile.

20. Procédé selon l'une des revendications 9 à 19 **caractérisé par le fait que** la quantité de base mise en oeuvre, exprimée par le rapport du nombre d'équivalents de OH- au nombre de moles de composé phénolique de formule (I) varie entre 0,5 et 3,0, et de préférence entre 1,0 et 2,0.

21. Procédé selon l'une des revendications 9 à 20 **caractérisé par le fait que** la quantité d'agent protecteur mise en oeuvre exprimée par le rapport entre le nombre de moles d'agent protecteur et le nombre de moles de composé phénolique de départ varie entre 1,0 et 1,5 et se situe de préférence, aux environs de 1,1.

22. Procédé selon l'une des revendications 9 à 21 **caractérisé par le fait que** la température à laquelle on réalise l'opération de protection du groupe hydroxyle du composé phénolique se situe entre 0°C et la température ambiante (le plus souvent entre 15°C et 25°C).

23. Procédé selon l'une des revendications 9 à 22 **caractérisé par le fait que** le composé phénolique sous forme protégée est mis en oeuvre tel quel dans l'opération suivante ou bien purifié par recristallisation dans un solvant organique, de préférence, l'éther éthylique.

24. Procédé selon la revendication 1 **caractérisé par le fait que** l'on fait réagir le composé phénolique sous forme protégée avec un réactif électrophile dans une réaction d'amidoalkylation, de Friedel-Crafts telle que notamment acylation, sulfonylation, alkylation, une réaction d'aminoalkylation, d'hydroxyalkylation, de formylation, de carboxylation et autre réaction de substitution électrophile.

25. Procédé selon la revendication 24 **caractérisé par le fait que** l'on fait réagir un composé phénolique protégé répondant préférentiellement à la formule (II), en présence d'un acide fort, avec un amide ou un carbamate et un composé carbonylé répondant plus particulièrement à la formule générale (V) : 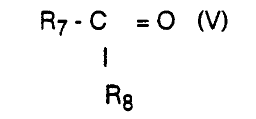 dans ladite formule (V) :
- R₇ représente un atome d'hydrogène,
- R₈ représente un atome d'hydrogène, un radical alkyle linéaire ou ramifié ayant de 1 à 6 atomes de carbone, un radical cycloalkyle ayant de 3 à 8 atomes de carbone, un radical alcényle ayant de 2 à 6 atomes de carbone ou un radical phényle éventuellement substitué ou un groupe électro-attracteur.

26. Procédé selon la revendication 25 **caractérisé par le fait que** le groupe électro-attracteur est :
- un groupe -CHO,
- un groupe acyle R₉-CO- ou R₉-CO-(CH₂)ₘ- dans lequel R₉ représente un radical alkyle linéaire ou ramifié ayant de 1 à 6 atomes de carbone et m représente un nombre de 1 à 3,
- un groupe -COOR₁₀ dans lequel R₁₀ représente un atome d'hydrogène ou un radical alkyle linéaire ou ramifié ayant de 1 à 6 atomes de carbone,
- un groupe -CX₂H dans lequel X représente un atome d'halogène, de préférence, un atome de fluor, chlore ou brome,
- un groupe -CX₃ dans lequel X représente un atome d'halogène, de préférence, un atome de fluor, chlore ou brome
- un groupe nitro.

27. Procédé selon l'une des revendications 25 et 26 **caractérisé par le fait que** le composé carbonylé répondant à la formule (V) est le formaldéhyde ou un générateur de formaldéhyde ; le glyoxal, l'acide glyoxylique, le bromal, le fluoral ; de préférence, le paraformaldéhyde.

28. Procédé selon la revendication 25 **caractérisé par le fait que** l'amide ou le carbamate mis en oeuvre répond la formule (VI) suivante : 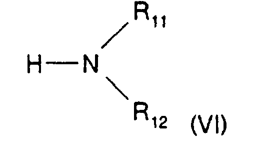 dans laquelle :
- au moins l'un des groupes R₁₁ et R₁₂, représente un groupe : 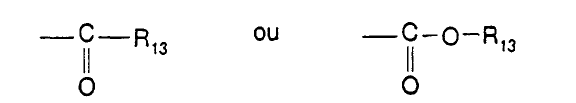
- les groupes R₁₁, R₁₂ et R₁₃, identiques ou différents, représentent :
. un atome d'hydrogène,
. un radical alkyle linéaire ou ramifié ayant de 1 à 12 atomes de carbone éventuellement porteur d'atomes d'halogène,
. un radical cycloalkyle ayant de 3 à 8 atomes de carbone, de préférence, un radical cyclopentyle, un radical cyclohexyle, un radical cycloheptyle
. un radical cycloalkylalkyle, de préférence, cyclopentylalkyle, cyclohexylalkyle, cycloheptylalkyle dont la partie alkyle comporte 1 à 4 atomes de carbone,
. un radical alkylaryle, de préférence, un radical phénylalkyle, dont la partie alkyle comporte 1 à 4 atomes de carbone, de préférence, un radical benzyle,
- deux groupes R₁₁ et R₁₂ peuvent former avec les deux atomes qui les portent, un cycle carbocyclique, saturé, insaturé ou aromatique ayant de 3 à 8 atomes de carbone.

29. Procédé selon la revendication 28 **caractérisé par le fait que** le composé de formule (VI) est l'acétamide, le chloroacétamide, le benzamide, le carbamate de benzyle.

30. Procédé selon l'une des revendications 25 à 29 **caractérisé par le fait que** l'on conduit la réaction de l'amidoalkylation du composé phénolique protégé, en présence d'un acide protonique ayant un pKa inférieur ou égal à 4,00, de préférence, inférieur ou égal à 3,00.

31. Procédé selon la revendication 30 **caractérisé par le fait que** l'acide est choisi parmi les oxacides minéraux halogénés ou non, de préférence, l'acide sulfurique ; les acides phosphoriques ; les acides phosphoniques ; les acides carboxyliques perhalogénés ou non ; les acides sulfoniques halogénés.

32. Procédé selon l'une des revendications 25 à 31 **caractérisé par le fait que** l'on conduit la réaction dans un solvant organique, de préférence, un acide aliphatique carboxylique.

33. Procédé selon la revendication 32 **caractérisé par le fait que** l'acide alphatique carboxylique est un acide carboxylique aliphatique saturé de préférence, l'acide acétique, l'acide propionique, l'acide butyrique, l'acide isobutyrique, l'acide pentanoïque, l'acide 2-méthylbutanoique.

34. Procédé selon l'une des revendications 25 à 33 **caractérisé par le fait que** le rapport entre le nombre de moles de composé carbonylé et le nombre de moles de composé phénolique protégé est d'au moins 1, de préférence, entre 1,0 et 2,0.

35. Procédé selon l'une des revendications 25 à 34 **caractérisé par le fait que** le rapport entre le nombre de moles d'amide ou de carbamate et le nombre de moles de composé carbonylé est choisi entre 1,0 et 3,0 et de préférence, aux environs de 2,0.

36. Procédé selon l'une des revendications 25 à 35 **caractérisé par le fait que** la quantité d'acide fort mise en oeuvre est telle que le rapport molaire H⁺/composé phénolique protégé varie entre 1,0 et 20 et de préférence entre 2,0 et 3,0.

37. Procédé selon l'une des revendications 25 à 36 **caractérisé par le fait que** la réaction est conduite avantageusement entre la température ambiante et 120°C, de préférence, entre 40°C et 80°C.

38. Procédé selon l'une des revendications 1 à 37 **caractérisé par le fait que** l'on soumet le produit amidoalkylé dont la fonction hydroxyle est protégée à un traitement à l'aide d'une base mise en oeuvre en une quantité telle que le pH se situe entre 7 et 10 afin d'effectuer le clivage du groupe sulfonyle et de libérer le groupe hydroxyle ce qui permet d'obtenir un composé phénolique amidoalkylé dont le groupe amidoalkyle est en position para par rapport au groupe électro-donneur.

39. Procédé selon l'une des revendications 1 à 38 **caractérisé par le fait que** le produit amidoalkylé dont la fonction hydroxyle est protégée est mis à réagir avec une solution acide, de préférence, une solution d'acide chlorhydrique ce qui permet d'obtenir un composé phénolique protégé et aminoalkylé dont le groupe aminoalkyle est en position para par rapport au groupe électro-donneur.

40. Procédé selon la revendication 24 **caractérisé par le fait que** l'on fait réagir le composé phénolique protégé répondant préférentiellement à la formule (II) avec un réactif électrophile, en présence d'un catalyseur de Friedel-Crafts.

41. Procédé selon la revendication 40 **caractérisé par le fait que** l'on fait appel à titre de réactifs électrophiles, aux acides carboxyliques et leurs dérivés, halogénures ou anhydrides, de préférence, anhydrides ; aux halogénures ou anhydrides de sulfonyle ou d'aminosulfonyle ; aux agents alkylants de type halogénure ou alcool.

42. Procédé selon l'une des revendications 40 et 41 **caractérisé par le fait que** les réactifs d'acylation ou de sulfonylation répondent plus particulièrement aux formules suivantes : 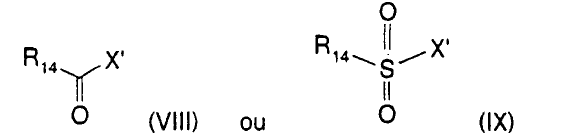
. dans les formules (VIII) ou (IX) :
- R₁₄ représente un radical aliphatique saturé ou insaturé, linéaire ou ramifié ayant de 1 à 24 atomes de carbone ; un radical cycloaliphatique, saturé, insaturé ou aromatique, monocyclique ou polycyclique, ayant de 4 à 12 atomes de carbone ; un radical aliphatique saturé ou insaturé, linéaire ou ramifié, porteur d'un substituant cyclique,
X' représente un atome d'halogène, de préférence un atome de chlore ou de brome,
. dans la formule (VIII) :
- X' représente un radical -O-CO-R₁₅ avec R₁₅, identique ou différent de R₁₄, ayant la même signification que R₁₄,
. dans la formule (IX) :
- X' représente un radical -O-SO₂-R₁₅ avec R₁₅, identique ou différent de R₁₄, ayant la même signification que R₁₄,
- R₁₄ représente :
. un radical alkoxy R₁₆-O- avec R₁₆ ayant la même signification que R₁₄,
. un groupe amino (R₁₇)(R₁₈)-N- avec R₁₇, identique ou différent de R₁₈, ayant la même signification que R₁₄.

43. Procédé selon l'une des revendications 40 et 41 **caractérisé par le fait que** le réactif d'alkylation répond à la formule générale (X) :
R₁₈-X (X)
dans ladite formule (X) :
- X symbolise un groupe hydroxyle ou un atome d'halogène : lesdits groupe hydroxyle ou atome d'halogène ne devant pas être en position vinylique ou alcynyle,
- R₁₈, éventuellement substitué, représente un radical aliphatique acyclique, saturé ou insaturé, linéaire ou ramifié ; un radical cycloaliphatique, saturé ou insaturé, monocylique ou polycyclique ; un radical aliphatique saturé ou insaturé, linéaire ou ramifié, porteur d'un substituant cyclique.

44. Procédé selon la revendication 24 **caractérisé par le fait que** l'on fait réagir le composé phénolique protégé répondant préférentiellement à la formule (II) avec un composé carbonylé, en présence d'une amine secondaire.

45. Procédé selon la revendication 44 **caractérisé par le fait que** le composé carbonylé répondant à la formule générale (XI) : 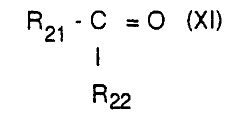 dans ladite formule (XI) :
- R₂₁ représente un atome d'hydrogène, un radical alkyle linéaire ou ramifié ayant de 1 à 6 atomes de carbone, un radical alcényle ayant de 2 à 6 atomes de carbone ou un radical phényle éventuellement substitué,
- R₂₂ peut être égal à R₂₁ ou représenter un groupe électro-attracteur.

46. Procédé selon la revendication 44 **caractérisé par le fait que** l'amine secondaire répond à la formule (XII) suivante : 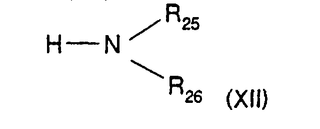 dans laquelle :
- R₂₅ et R₂₆, identiques ou différents, représentent :
. un radical alkyle linéaire ayant de 1 à 12 atomes de carbone, un radical alkyle secondaire ayant 3 à 12 atomes de carbone ou un radical alkyle tertiaire ayant 4 à 12 atomes de carbone, ces radicaux alkyles pouvant comporter une ou deux fonctions éther - O - ou fonctions hydroxyle, amine tertiaire, acide carboxylique, ester d'acide carboxylique,
. un radical phényle, un radical cyclohexyle, un radical cycloheptyle ou un radical cyclopentyle,
. un radical phénylalkyle, cyclohexylalkyle, cycloheptylalkyle ou cyclopentylalkyle dont la partie alkyle comporte 1 à 4 atomes de carbone,
. un atome d'hydrogène,
- R₂₅ ou R₂₆ forment ensemble et avec l'atome d'azote un hétérocycle, saturé ou comportant une ou plusieurs doubles liaisons, éventuellement substitué par un ou plusieurs radicaux alkyles ayant 1 à 4 atomes de carbone ;
- R₂₅ et R₂₆ forment ensemble, avec l'atome d'azote et avec un ou plusieurs atomes d'azote et/ou d'oxygène et/ou de soufre, un hétérocycle, saturé ou insaturé, éventuellement substitué par un ou plusieurs radicaux alkyles ayant 1 à 4 atomes de carbone ;
- R₂₅ et R₂₆ forment ensemble et avec l'atome d'azote un hétérocycle insaturé éventuellement substitué par un deux groupements méthyle ou éthyle ;
- R₂₅ et R₂₆ forment ensemble, avec l'atome d'azote et éventuellement avec un ou plusieurs atomes d'azote et/ou d'oxygène et/ou de soufre, un composé polycyclique, saturé ou insaturé, éventuellement substitué par un ou plusieurs radicaux alkyles ayant 1 à 4 atomes de carbone.

47. Procédé selon la revendication 44 **caractérisé par le fait que** l'on fait réagir le composé carbonylé puis l'amine secondaire afin de former le réactif hémiaminal, puis le composé phénolique protégé et le catalyseur acide.

48. Procédé selon la revendication 24 **caractérisé par le fait que** l'on fait réagir le composé phénolique protégé répondant préférentiellement à la formule (II) avec un composé carbonylé, en présence d'un catalyseur de type de Brônsted ou une zéolithe.

49. Procédé selon la revendication 48 **caractérisé par le fait que** le composé carbonylé répond à la formule générale (XI) définie dans la revendication 45.

50. Procédé selon la revendication 24 **caractérisé par le fait que** l'on fait réagir le composé phénolique protégé répondant préférentiellement à la formule (II) avec le chloral ou l'hexaméthylènetétramine.

51. Procédé selon la revendication 50 **caractérisé par le fait que** la réaction est conduite dans un acide de préférence l'acide trifluroroacétique, l'acide sulfurique, l'acide fluorhydrique, l'acide borique.

52. Procédé selon l'une des revendications 40 à 51 **caractérisé par le fait que** l'on fait suivre l'étape de substitution électrophile par une étape de déprotection du groupe hydroxyle, de préférence, en mettant en oeuvre le mode décrit dans la revendication 38.

## Patentansprüche

1. Verfahren zur Funktionalisierung in para-Position zu einer an eine Phenolverbindung gebundene Elektronendonorgruppe, das einen ersten Schritt des Schützens der Hydroxylgruppe, einen zweiten Schritt des Umsetzens der geschützten Phenolverbindung mit einem elektrophilen Reagens, gegebenenfalls einen dritten Schritt des Entschützens der Hydroxylgruppe umfasst, **dadurch gekennzeichnet, dass** der Schutz der Hydroxylgruppe einer Phenolverbindung, die eine Elektronendonorgruppe trägt, in Form einer Sulfonesterfunktion durchgerührt wird.

2. Verfahren nach Anspruch 1, **dadurch gekennzeichnet, dass** die phenolische Ausgangsverbindung Träger mindestens einer Elektronendonorgruppe in ortho-Position zu der Hydroxylgruppe und eines Wasserstoffatoms in para-Position der Elektronendonorgruppe ist.

3. Verfahren nach einem der Ansprüche 1 und 2, **dadurch gekennzeichnet, dass** die phenolische Ausgangsverbindung der allgemeinen Formel (I) entspricht: 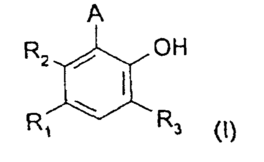 wobei in der Formel (I):
- A eine Elektronendonorgruppe darstellt,
- R₁, R₂ und R₃, gleich oder verschieden, folgendes darstellen:
- ein Wasserstoffatom,
- einen linearen oder verzweigten Alkylrest mit 1 bis 20 Kohlenstoffatomen, der gegebenenfalls ein oder mehrere Halogenatome trägt,
- eine Cycloalkylgruppe mit 3 bis 8 Kohlenstoffatomen und vorzugsweise 6 Kohlenstoffatomen,
- einen Aralkylrest mit 6 bis 20 Kohlenstoffatomen,
- einen Arylrest mit 6 bis 20 Kohlenstoffatomen,
- ein Halogenatom,
- eine elektronenziehende Gruppe,
- zwei Gruppen R₁ und R₂ mit den beiden Atomen, an die sie gebunden sind, einen gesättigten, ungesättigten oder aromatischen carbocyclischen Ring mit 3 bis 8 Kohlenstoffatomen bilden können.

4. Verfahren nach Anspruch 3, **dadurch gekennzeichnet, dass** die elektronenziehende Gruppe eine Carboxyl- oder Estergruppe (vorzugsweise mit 3 bis 8 Kohlenstoffatomen) oder eine Nitril- oder Nitrogruppe ist.

5. Verfahren nach einem der Ansprüche 1 bis 4, **dadurch gekennzeichnet, dass** die phenolische Ausgangsverbindung der Formel (I) entspricht, wobei R₁, R₂ und R₃ ein Wasserstoffatom, eine lineare oder verzweigte Alkylgruppe mit 1 bis 4 Kohlenstoffatomen, ein Halogenatom oder eine Trifluormethylgruppe darstellen.

6. Verfahren nach einem der Ansprüche 1 bis 5, **dadurch gekennzeichnet, dass** die phenolische Ausgangsverbindung der Formel (I) entspricht, wobei A eine der folgenden Gruppen oder Funktionen darstellt:
- eine lineare oder verzweigte Alkylgruppe mit vorzugsweise 1 bis 6 Kohlenstoffatomen und mehr bevorzugt mit 1 bis 4 Kohlenstoffatomen,
- eine Cycloalkylgruppe mit 3 bis 8 Kohlenstoffatomen und vorzugsweise 6 Kohlenstoffatomen,
- einen Phenylrest,
- einen Benzyl- oder Phenylethylrest,
- eine Hydroxylgruppe,
- ein Fluoratom,
- eine Alkoxygruppe mit 1 bis 6 Kohlenstoffatomen, mehr bevorzugt mit 1 bis 4 Kohlenstoffatomen im Alkylteil oder eine Phenoxygruppe,
- eine Aminogruppe, vorzugsweise disubstituiert, wobei die Substituenten, gleich oder verschieden, lineare oder verzweigte Alkylreste mit 1 bis 6 Kohlenstoffatomen und mehr bevorzugt mit 1 bis 4 Kohlenstoffatomen oder Phenyl sind,
- eine Alkylamid- oder Arylamidgruppe, wobei die Alkylgruppe 1 bis 6 Kohlenstoffatome und vorzugsweise 1 bis 4 Kohlenstoffatome aufweist und die Arylgruppe 6 bis 12 Kohlenstoffatome aufweist und vorzugsweise eine Phenylgruppe ist.

7. Verfahren nach einem der Ansprüche 1 bis 6, **dadurch gekennzeichnet, dass** die phenolische Ausgangsverbindung der Formel (I) entspricht, wobei A eine Methoxy- oder Ethoxygruppe darstellt.

8. Verfahren nach einem der Ansprüche 1 bis 7, **dadurch gekennzeichnet, dass** die phenolische Ausgangsverbindung ausgewählt ist aus:
- Brenzcatechin,
- Resorcin,
- o-Cresol,
- m-Cresol,
- 2-Ethylphenol,
- 3-Ethylphenol,
- 2-Propylphenol,
- 2-sek-Butylphenol,
- 2-tert-Butylphenol,
- 3-tert-Butylphenol,
- 2-Methoxyphenol,
- 3-Mehtoxyphenol,
- 2-Ethoxyphenol,
- 3-Ethoxyphenol,
- 2-Chlorphenol,
- 3-Chlorphenol,
- 2,3-Dimethylphenol,
- 2,5-Dimethylphenol,
- 2,6-Dimethylphenol,
- 3,5-Dimethylphenol,
- Vanillin,
- 2,3-Dichlorphenol,
- 2,6-Dichlorphenol,
- 3,5-Dichlorphenol,
- Pyrogallol,
- 2,3,5-Trimethylphenol,
- 2,3,6-Trimethylphenol,
- 2,6-di-tert-Butylphenol,
- 3,5-di-tert-Butylphenol,
- 2,3,6-Trichlorphenol,
- 1,2-Dihydroxynaphthalin,
- 1,4-Dihydroxynaphthalin,
- 1,5-Dihydroxynaphthalin,
- 2,3-Dihydroxynaphthalin,
- 2,6-Dihydroxynaphthalin,
- 2,7-Dihydroxynaphthalin,
- 4-Methoxy-1-naphthol,
- 6-Brom-2-naphthol,
- 2-Phenoxyphenol,
- 3-Phenoxyphenol.

9. Verfahren nach Anspruch 1, **dadurch gekennzeichnet, dass** in einem ersten Schritt die Hydroxylfunktion durch Überführen in Sulfonester geschützt wird.

10. Verfahren nach Anspruch 9, **dadurch gekennzeichnet, dass** die bevorzugten Sulfonestergruppen folgende sind:
- Tosylate (p-Toluolsulfonate) -OSO₂-C₆H₄-CH₃
- Brosylate (p-Brombenzolsulfonate) -OSO₂-C₆H₄-Br
- Nosylate (p-Nitrobenzolsulfonate) -OSO₂-C₆H₄-NO₂
- Mesylate (Methansulfonate) -OSO₂-CH₃
- Betylate (Ammoniumalkansulfonate) -OSO₂-(CH₂)ₙNNe₃⁺, wobei n 0 bis 6 ist,
- Triflate (Trifluormethansulfonate) -OSO₂-CF₃
- Nonaflate (Nonafluorbutansulfonate) -OSO₂-C₄F₉
- Tresylate (2,2,2-Trifluorethansulfonate) -OSO₂-CH₂-CF₃.

11. Verfahren nach einem der Ansprüche 1 bis 10, **dadurch gekennzeichnet, dass** die Phenolverbindung in geschützter Form der allgemeinen Formel (II) entspricht: 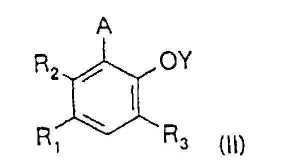 wobei in der Formel (II):
- Y die folgende Gruppe darstellt: 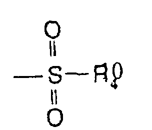 wobei R₄ einen Kohlenwasserstoffrest mit 1 bis 20 Kohlenstoffatomen darstellt und insbesondere folgendes darstellt:
- einen Alkylrest mit 1 bis 10 Kohlenstoffatomen, vorzugsweise 1 bis 4 Kohlenstoffatomen und mehr bevorzugt einen Methyl- oder Ethylrest, der gegebenenfalls ein Halogenatom, eine Gruppe CF₃ oder eine Ammoniumgruppe N(R₅)₄ trägt, wobei R₅, gleich oder verschieden, einen Alkylrest mit 1 bis 4 Kohlenstoffatomen darstellt,
- einen Cycloalkylrest mit 3 bis 8 Kohlenstoffatomen, vorzugsweise einen Cyclohexylrest,
- einen Arylrest mit 6 bis 12 Kohlenstoffatomen, vorzugsweise einen Phenylrest, der gegebenenfalls einen Alkylrest mit 1 bis 10 Kohlenstoffatomen, vorzugsweise 1 bis 4 Kohlenstoffatomen, und mehr bevorzugt einen Methyl- oder Ethylrest, ein Halogenatom, eine Gruppe CF₃ oder eine Gruppe NO₂ trägt,
- eine Gruppe CX₃, wobei X ein Fluor-, Chlor- oder Bromatom darstellt,
- eine Gruppe CF₂-CF₃.
- A, R₁, R₂ und R₃ die bereits zuvor angegebene Bedeutung besitzen.

12. Verfahren nach Anspruch 11, **dadurch gekennzeichnet, dass** das geschützte Phenol der Formel (II) durch Umsetzung in Gegenwart einer Base der phenolischen Ausgangsverbindung der Formel (I) mit einem Schutzmittel, das der folgenden Formel (III) entspricht: 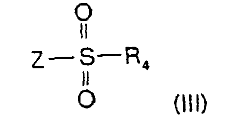 wobei in der Formel (III):
- R₄ die bereits zuvor gegebene Bedeutung besitzt,
- Z folgendes darstellt:
- eine Hydroxylgruppe oder ein Halogenatom, vorzugsweise ein Chlor- oder Bromatom,
- eine Gruppe -O-SO₂-R₄^{'}, wobei R₄^{'}, gleich oder verschieden von R₄, die für R₄ angegebene Bedeutung besitzt,
erhalten wird.

13. Verfahren nach Anspruch 12, **dadurch gekennzeichnet, dass** das Schutzmittel der Formel (III) entspricht, wobei Z ein Chlor- oder Bromatom darstellt.

14. Verfahren nach einem der Ansprüche 12 und 13, **dadurch gekennzeichnet, dass** das Schutzmittel ausgewählt ist aus:
- Triflatanhydrid,
- Methansulfonylchlorid,
- Benzolsulfonylchlorid,
- p-Toluolsulfonylchlorid.

15. Verfahren nach einem der Ansprüche 9 bis 14, **dadurch gekennzeichnet, dass** eine mineralische oder organische Base verwendet wird.

16. Verfahren nach Anspruch 15, **dadurch gekennzeichnet, dass** die verwendete Base ein Alkalimetallhydroxid, vorzugsweise Natrium- oder Kaliumhydroxid; ein Carbonat, ein Bicarbonat, ein Phosphat oder ein Hydrogenphosphat, ein Acetat, ein Alkalimetalltrifluoracetat, vorzugsweise Natrium- oder Kaliumtrifluoracetat; ein tertiäres Amin ist.

17. Verfahren nach Anspruch 16, **dadurch gekennzeichnet, dass** das tertiäre Amin der allgemeinen Formel (IV) entspricht:
N-(R₆)₃ (IV)
wobei:
- die Gruppen R₆, gleich oder verschieden, Kohlenwasserstoffreste mit 1 bis 20 Kohlenstoffatomen darstellen, wie Alkyl-, Cycloalkyl-, Aryl- oder heterocyclische Gruppen;
- 2 Gruppen R₆ zusammen und mit dem Stickstoffatom einen Heterocyclus mit 4 bis 5 Atomen bilden.

18. Verfahren nach Anspruch 17, **dadurch gekennzeichnet, dass** das Amin Triethylamin, Tri-*n*-propylamin, tri-*n*-butylamin, Methyldibutylamin, Methyldicyclohexylamin, Ethyldiisopropylamin, N,N-Diethylcyclohexylamin, Dimethylamino-4-pyridin, N-Methylpiperidin, N-Ethylpiperidin, N-*n*-Butylpiperidin, 1,2-Dimethylpiperidin, N-Methylpyrrolidin, 1,2-Dimethylpyrrolidin ist.

19. Verfahren nach einem der Ansprüche 9 bis 18, **dadurch gekennzeichnet, dass** die Reaktion zum Schutze der Phenolverbindung in einem organischen polaren oder apolaren Lösungsmittel, vorzugsweise einem aliphatischen, cycloaliphatischen, halogenierten oder nicht halogenierten Kohlenwasserstoff; einem Ester; einem Ether oder Nitril durchgeführt wird.

20. Verfahren nach einem der Ansprüche 9 bis 19, **dadurch gekennzeichnet, dass** die Menge der verwendeten Base, ausgedrückt durch das Verhältnis der Anzahl von OH⁻-Äquivalenten zur Anzahl von Molen an Phenolverbindung der Formel (I) zwischen 0,5 und 3,0 und vorzugsweise zwischen 1,0 und 2,0 schwankt.

21. Verfahren nach einem der Ansprüche 9 bis 20, **dadurch gekennzeichnet, dass** die Menge an verwendetem Schutzmittel, ausgedrückt durch das Verhältnis zwischen der Anzahl von Molen an Schutzmittel und der Anzahl von Molen an phenolischer Ausgangsverbindung zwischen 1,0 und 1,5 schwankt und vorzugsweise bei etwa 1,1 liegt.

22. Verfahren nach einem der Ansprüche 9 bis 21, **dadurch gekennzeichnet, dass** die Temperatur, bei der der Vorgang des Schutzes der Hydroxylgruppe der Phenolverbindung durchgeführt wird, zwischen 0 °C und der Umgebungstemperatur (häufiger zwischen 15 °C und 25 °C) liegt.

23. Verfahren nach einem der Ansprüche 9 bis 22, **dadurch gekennzeichnet, dass** die phenolische Verbindung in geschützter Form bei dem folgenden Vorgang verwendet wird, wie sie ist oder durch Umkristallisation in einem organischen Lösungsmittel, vorzugsweise Ethylether, gereinigt verwendet wird.

24. Verfahren nach Anspruch 1, **dadurch gekennzeichnet, dass** die phenolische Verbindung in geschützter Form mit einem elektrophilen Reagens in einer Friedel-Craft-Amidoalkylierungsreaktion, wie insbesondere eine Acylierung, Sulfonylierung, Alkylierung, eine Aminoalkylierungsreaktion, eine Hydroxyalkylierungsreaktion, Formylierungsreaktion, Carboxylierungsreaktion und eine andere elektrophile Substitutionsreaktion, reagieren gelassen wird.

25. Verfahren nach Anspruch 24, **dadurch gekennzeichnet, dass** eine geschützte phenolische Verbindung, die vorzugsweise der Formel (II) entspricht, in Gegenwart einer starken Säure mit einem Amid oder einem Carbamat und einer Carbonylverbindung reagieren gelassen wird, die insbesondere der allgemeinen Formel (V) entspricht: 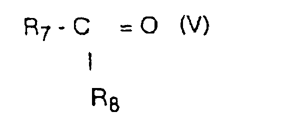 wobei in der Formel (V):
- R₇ ein Wasserstoffatom darstellt,
- R₈ ein Wasserstoffatom, einen linearen oder verzweigten Alkylrest mit 1 bis 6 Kohlenstoffatomen, einen Cycloalkylrest mit 3 bis 8 Kohlenstoffatomen, einen Alkenylrest mit 2 bis 6 Kohlenstoffatomen oder einen Phenylrest, der gegebenenfalls substituiert ist, oder eine elektronenziehende Gruppe darstellt.

26. Verfahren nach Anspruch 25, **dadurch gekennzeichnet, dass** die elektronenziehende Gruppe folgende ist:
- eine Gruppe -CHO,
- eine Acylgruppe R₉-CO- oder R₉-CO-(CH₂)ₘ-, wobei R₉ einen linearen oder verzweigten Alkylrest mit 1 bis 6 Kohlenstoffatomen darstellt und m eine Zahl von 1 bis 3 darstellt,
- eine Gruppe -COOR₁₀, wobei R₁₀ ein Wasserstoffatom oder einen linearen oder verzweigten Alkylrest mit 1 bis 6 Kohlenstoffatomen darstellt,
- eine Gruppe -CX₂H, wobei X ein Halogenatom, vorzugsweise ein Fluor-, Chlor- oder Bromatom darstellt,
- eine Gruppe -CX₃, wobei X ein Halogenatom, vorzugsweise ein Fluor-, Chlor-oder Bromatom darstellt,
- eine Nitrogruppe.

27. Verfahren nach einem der Ansprüche 25 und 26, **dadurch gekennzeichnet, dass** die Carbonylverbindung, die der Formel (V) entspricht, Formaldehyd oder ein Formaldehydbildner; Glyoxal, Glyoxylsäure, Bromal, Fluoral und vorzugsweise Paraformaldehyd ist.

28. Verfahren nach Anspruch 25, **dadurch gekennzeichnet, dass** das verwendete Amid oder Carbamat der folgenden Formel (VI) entspricht: 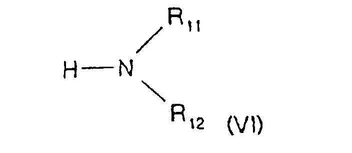 wobei:
- mindestens eine der Gruppen R₁₁ und R₁₂ eine Gruppe 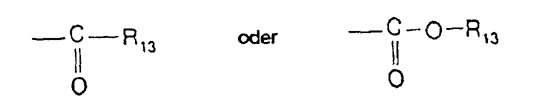 darstellen;
- die Gruppen R₁₁, R₁₂ und R₁₃, gleich oder verschieden, folgendes darstellen:
- ein Wasserstoffatom,
- einen linearen oder verzweigten Alkylrest mit 1 bis 12 Kohlenstoffatomen, der gegebenenfalls Halogenatome trägt,
- einen Cycloalkylrest mit 3 bis 8 Kohlenstoffatomen, vorzugsweise einen Cyclopentyl-, Cyclohexyl-, Cycloheptylrest,
- einen Cycloalkylalkylrest, vorzugsweise Cyclopentylalkyl, Cyclohexylalkyl, Cycloheptylalkyl, deren Alkylteil 1 bis 4 Kohlenstoffatomen umfasst,
- einen Alkylarylrest, vorzugsweise einen Phenylalkylrest, dessen Alkylteil 1 bis 4 Kohlenstoffatome umfasst, vorzugsweise einen Benzylrest,
- zwei Gruppen R₁₁ und R₁₂ mit den beiden Atomen, an die sie gebunden sind, einen carbocyclischen, gesättigten, ungesättigten oder aromatischen Ring mit 3 bis 8 Kohlenstoffatomen bilden können.

29. Verfahren nach Anspruch 28, **dadurch gekennzeichnet, dass** die Verbindung der Formel (VI) Acetamid, Chloracetamid, Benzamid, Benzylcarbamat ist.

30. Verfahren nach einem der Ansprüche 25 bis 29, **dadurch gekennzeichnet, dass** die Amidoalkylierungsreaktion der geschützten Phenolverbindung in Gegenwart einer Protonensäure, mit einem pKa-Wert von kleiner oder gleich 4,00, vorzugsweise von kleiner oder gleich 3,00 durchgeführt wird.

31. Verfahren nach Anspruch 30, **dadurch gekennzeichnet, dass** die Säure ausgewählt ist aus halogenierten oder nicht-halogenierten mineralischen Sauerstoffsäuren, vorzugsweise Schwefelsäure; Phosphorsäuren; Phosphonsäuren; perhalogenierten oder nichtperhalogenierten Carbonsäuren; halogenierten Sulfonsäuren.

32. Verfahren nach einem der Ansprüche 25 bis 31, **dadurch gekennzeichnet, dass** die Reaktion in einem organischen Lösungsmittel, vorzugsweise einer aliphatischen Carbonsäure durchgeführt wird.

33. Verfahren nach Anspruch 32, **dadurch gekennzeichnet, dass** die aliphatische Carbonsäure eine gesättigte, aliphatische Carbonsäure, vorzugsweise Essigsäure, Propionsäure, Butansäure, Isobutansäure, Pentansäure, 2-Methylbutansäure ist.

34. Verfahren nach einem der Ansprüche 25 bis 33, **dadurch gekennzeichnet, dass** das Verhältnis zwischen der Anzahl von Molen an Carbonylverbindung und der Anzahl von Molen an geschützter Phenolverbindung mindestens 1 ist, vorzugsweise zwischen 1,0 und 2,0 liegt.

35. Verfahren nach einem der Ansprüche 25 bis 34, **dadurch gekennzeichnet, dass** das Verhältnis zwischen der Anzahl von Molen an Amid oder Carbamat und der Anzahl von Molen an Carbonylverbindung zwischen 1,0 und 3,0 und vorzugsweise bei etwa 2,0 gewählt wird.

36. Verfahren nach einem der Ansprüche 25 bis 35, **dadurch gekennzeichnet, dass** die Menge an starker Säure, die verwendet wird, so ist, dass das Molverhältnis H⁺/geschützte Phenolverbindung zwischen 1,0 und 2,0 und vorzugsweise zwischen 2,0 und 3,0 schwankt.

37. Verfahren nach einem der Ansprüche 25 bis 36, **dadurch gekennzeichnet, dass** die Reaktion zweckmäßigerweise zwischen Umgebungstemperatur und 120 °C, vorzugsweise zwischen 40 °C und 80 °C durchgeführt wird.

38. Verfahren nach einem der Ansprüche 1 bis 37, **dadurch gekennzeichnet, dass** das amidoalkylierte Produkt, dessen Hydroxylfunktion geschützt ist, einer Behandlung mit einer Base, die in einer solchen Menge verwendet wird, dass der pH zwischen 7 und 10 liegt, unterzogen wird, um die Abspaltung der Sulfonylgruppe zu bewirken und die Hydroxylgruppe freizusetzen, wodurch sich eine amidoalkylierte Phenolverbindung erhalten lässt, deren Amidoalkylgruppe sich in para-Position zu der Elektronendonorgruppe befindet.

39. Verfahren nach einem der Ansprüche 1 bis 38, **dadurch gekennzeichnet, dass** das amidoalkylierte Produkt, dessen Hydroxylfunktion geschützt ist, mit einer Säurelösung vorzugsweise einer Chlorwasserstoffsäurelösung, reagieren gelassen wird, wodurch sich eine geschützte und aminoalkylierte Phenolverbindung erhalten lässt, deren Aminoalkylgruppe sich in para-Position zu der Elektronendonorgruppe befindet.

40. Verfahren nach Anspruch 24, **dadurch gekennzeichnet, dass** die geschützte Phenolverbindung, die vorzugsweise der Formel (II) entspricht, mit einem elektrophilen Reagens in Gegenwart eines Friedel-Craft-Katalysators reagieren gelassen wird.

41. Verfahren nach Anspruch 40, **dadurch gekennzeichnet, dass** als elektrophile Reagentien Carbonsäuren und ihre Halogen- oder Anhydridderivate, vorzugsweise die Anhydride; Sulfonyl- oder Aminosulfonylhalogenide oder -anhydride; Alkylierungsmittel vom halognierten oder alkoholischen Typ verwendet werden.

42. Verfahren nach einem der Ansprüche 40 und 41, **dadurch gekennzeichnet, dass** das Acylierungs- oder Sulfonylierungsreagens insbesondere den folgenden Formeln entspricht: 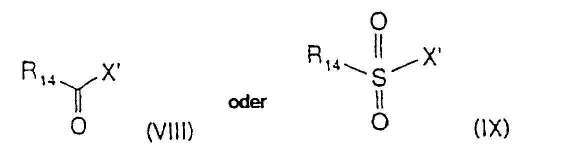 wobei
- in den Formeln (VIII) oder (IX):
- R₁₄ einen gesättigten oder ungesättigten, linearen oder verzweigten, aliphatischen Rest mit 1 bis 24 Kohlenstoffatomen; einen cycloaliphatischen, gesättigten, ungesättigten oder aromatischen mono- oder polycyclischen Rest mit 4 bis 12 Kohlenstoffatomen; einen aliphatischen, gesättigten oder ungesättigten, linearen oder verzweigten Rest, der einen cyclischen Substituenten trägt, darstellt,
X' ein Halogenatom, vorzugsweise ein Chlor- oder Bromatom darstellt,
- in der Formel (VIII):
- X' einen Rest -O-CO-R₁₅ darstellt, wobei R₁₅, gleich oder verschieden von R₁₄, die gleiche Bedeutung wie R₁₄ besitzt,
- in der Formel (IX):
- X' einen Rest -O-SO₂-R₁₅ darstellt, wobei R₁₅, gleich oder verschieden von R₁₄, die gleiche Bedeutung wie R₁₄ besitzt,
- R₁₄ folgendes darstellt:
- einen Alkoxyrest R₁₆-O-, wobei R₁₆ die gleiche Bedeutung wie R₁₄ aufweist,
- eine Aminogruppe (R₁₇)(R₁₈)-N-, wobei R₁₇, gleich oder verschieden von R₁₈, die gleiche Bedeutung wie R₁₄ besitzt.

43. Verfahren nach einem der Ansprüche 40 und 41, **dadurch gekennzeichnet, dass** das Alkylierungsreagens der allgemeinen Formel (X) entspricht:
R₁₈-X (X)
wobei in der Formel (X):
- X für eine Hydroxylgruppe oder ein Halogenatom steht; wobei diese, Hydroxylgruppe oder Halogenatom, nicht in vinylischer oder alkinylischer Position sein dürfen,
- R₁₈, gegebenenfalls substituiert, einen acyclischen, gesättigten oder ungesättigten, linearen oder verzweigten, aliphatischen Rest; einen cycloaliphatischen, gesättigten oder ungesättigten, monocyclischen oder polycyclischen Rest; einen gesättigten oder ungesättigten, linearen oder verzweigten, aliphatischen Rest, der einen cyclischen Substituenten trägt, darstellt.

44. Verfahren nach Anspruch 24, **dadurch gekennzeichnet, dass** die geschützte Phenolverbindung, die vorzugsweise der Formel (II) entspricht, mit einer Carbonylverbindung in Gegenwart eines sekundären Amins reagieren gelassen wird.

45. Verfahren nach Anspruch 44, **dadurch gekennzeichnet, dass** die Carbonylverbindung der allgemeinen Formel (XI) entspricht: 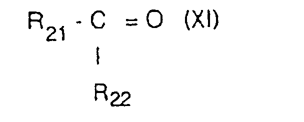 wobei in der Formel (XI):
- R₂₁ ein Wasserstoffatom, einen linearen oder verzweigten Alkylrest mit 1 bis 6 Kohlenstoffatomen, einen Alkenylrest mit 2 bis 6 Kohlenstoffatomen oder einen gegebenenfalls substituierten Phenylrest darstellt,
- R₂₂ gleich R₂₁ sein kann oder eine elektronenziehende Gruppe darstellen kann.

46. Verfahren nach Anspruch 44, **dadurch gekennzeichnet, dass** das sekundäre Amin der folgenden Formel (XII) entspricht: 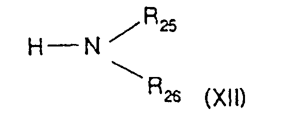 wobei:
- R₂₅ und R₂₆, gleich oder verschieden, folgendes darstellen:
- einen linearen Alkylrest mit 1 bis 12 Kohlenstoffatomen, einen sekundären Alkylrest mit 3 bis 12 Kohlenstoffatomen oder einen tertiären Alkylrest mit 4 bis 12 Kohlenstoffatomen, wobei diese Alkylreste eine oder zwei Etherfunktionen -O- oder Hydroxyl-, tertiäre Amin-, Carbonsäure-, Carbonsäureesterfunktionen umfassen können,
- einen Phenylrest, einen Cyclohexylrest, einen Cycloheptylrest oder einen Cyclopentylrest,
- einen Phenylalkylrest, Cyclohexylalkylrest, Cycloheptylalkylrest oder Cyclopentylalkylrest, dessen Alkylteil 1 bis 4 Kohlenstoffatome umfasst,
- ein Wasserstoffatom,
- R₂₅ oder R₂₆ zusammen mit dem Stickstoffatom einen gesättigten Heterocyclus bilden oder eine oder mehrere Doppelbindungen, gegebenenfalls mit einem oder mehreren Alkylresten mit 1 bis 4 Kohlenstoffatomen substituiert, umfassen;
- R₂₅ und R₂₆ zusammen mit dem Stickstoffatom und mit einem oder mehreren Stickstoff- und/oder Sauerstoff- und/oder Schwefelatomen einen gesättigten oder ungesättigten Heterozyklus, gegebenenfalls mit einem oder mehreren Alkylresten, mit 1 bis 4 Kohlenstoffatomen substituiert, bilden;
- R₂₅ und R₂₆ zusammen mit dem Stickstoffatom einen ungesättigten Heterocyclus, gegebenenfalls mit einer oder zwei Methyl- oder Ethylgruppierungen substituiert, bilden;
- R₂₅ und R₂₆ zusammen mit dem Stickstoffatom und gegebenenfalls mit einem oder mehreren Stickstoff- und/oder Sauerstoff- und/oder Schwefelatomen eine gesättigte oder ungesättigte polycyclische Verbindung, gegebenenfalls mit einem oder mehreren Alkylresten mit 1 bis 4 Kohlenstoffatomen substituiert, bilden.

47. Verfahren nach Anspruch 44, **dadurch gekennzeichnet, dass** die Carbonylverbindung und das sekundäre Amin unter Bildung des Hemiaminalreagens und anschließend die geschütztz Phenolverbindung und der Säurekatalysator reagieren gelassen werden.

48. Verfahren nach Anspruch 24, **dadurch gekennzeichnet, dass** die geschützte Phenolverbindung, die vorzugsweise der Formel (II) entspricht, mit einer Carbonylverbindung in Gegenwart eines Katalysators vom Brönsted-Typ oder mit einem Zeolith reagieren gelassen wird.

49. Verfahren nach Anspruch 48, **dadurch gekennzeichnet, dass** die carbonylierte Verbindung der allgemeinen Formel (XI) entspricht, die in Anspruch 45 definiert ist.

50. Verfahren nach Anspruch 24, **dadurch gekennzeichnet, dass** die geschützte Phenolverbindung, die vorzugsweise der Formel (II) entspricht, mit Chloral oder Hexamethylentetramin reagieren gelassen wird.

51. Verfahren nach Anspruch 50, **dadurch gekennzeichnet, dass** die Reaktion in einer Säure, vorzugsweise Trifluoressigsäure, Schwefelsäure, Fluorwasserstoffsäure, Borsäure durchgeführt wird.

52. Verfahren nach einem der Anspruch 40 bis 51, **dadurch gekennzeichnet, dass** man dem Schritt der elektrophilen Substitution einen Schritt der Entschützung der Hydroxylgruppe, vorzugsweise unter Durchführung der in Anspruch 38 beschriebenen Weise, folgen lässt.

## Claims

1. A process for functionalisation in the position para with respect to an electron-donating group carried by a phenolic compound, comprising a first step for protecting the hydroxy group, a second step for reacting the protected phenolic compound with an electrophilic reactant, and optionally, a third step for deprotecting the hydroxy group, **characterized in that** the hydroxy group of the phenolic compound carrying an electron-donating group is protected by means of a sulphonic ester function.

2. A process according to claim 1, **characterized in that** the starting phenolic compound carries at least one electron-donating group in the position ortho to the hydroxy group and a hydrogen atom in the position para to the electron-donating group.

3. A process according to claim 1 or claim 2, **characterized in that** the starting phenolic compound has general formula (I): 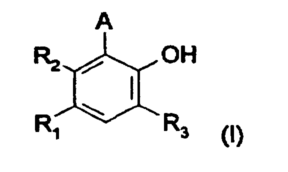 in which formula (I):
• A represents an electron-donating group;
• R₁, R₂ and R₃, which may be identical or different, represent:
• a hydrogen atom;
• a linear or branched alkyl radical containing 1 to 20 carbon atoms, optionally carrying one or more halogen atoms;
• a cycloalkyl group containing 3 to 8 carbon atoms, preferably 6 carbon atoms;
• an aralkyl radical containing 6 to 20 carbon atoms;
• an aryl radical containing 6 to 20 carbon atoms;
• a halogen atom;
• an electron-attracting group;
• two groups R₁ and R₂ together with the two atoms carrying them, form a saturated, unsaturated or aromatic carbocyclic cycle containing 3 to 8 carbon atoms.

4. A process according to claim 3, **characterized in that** the electron-attracting group is a carboxy group or an ester group (preferably containing 3 to 8 carbon atoms) or a nitrile or a nitro group.

5. A process according to any one of claims 1 to 4, **characterized in that** the startingphenolic compound has formula (I) in which R₁, R₂ and R₃ represent a hydrogen atom, a linear or branched alkyl group containing 1 to 4 carbon atoms, a halogen atom or a trifluoromethyl group.

6. A process according to any one of claims 1 to 5, **characterized in that** the starting phenolic compound has formula (I) in which A represents one of the following groups or functions:
• a linear or branched alkyl group preferably containing 1 to 6 carbon atoms, more preferably containing 1 to 4 carbon atoms;
• a cycloalkyl group containing 3 to 8 carbon atoms, preferably 6 carbon atoms;
• the phenyl radical;
• the benzyl or phenylethyl radical;
• the hydroxy group;
• a fluorine atom;
• an alkoxy group containing 1 to 6 carbon atoms, more preferably 1 to 4 carbon atoms in the alkyl portion, or a phenoxy group;
• an amino group, preferably di-substituted in which the substituents, which may be identical or different, are linear or branched alkyl radicals containing 1 to 6 carbon atoms, more preferably 1 to 4 carbon atoms, or a phenyl group;
• an alkylamide or arylamide group in which the alkyl group contains 1 to 6 carbon atoms, preferably 1 to 4 carbon atoms, and the aryl group contains 6 to 12 carbon atoms and is preferably a phenyl group.

7. A process according to any one of claims 1 to 6, **characterized in that** the starting phenolic compound has formula (I) in which A represents a methoxy or an ethoxy group.

8. A process according to any one of claims 1 to 7, **characterized in that** the starting phenolic compound is selected from:
• pyrocatechin;
• resorcin;
• o-cresol;
• m-cresol;
• 2-ethylphenol;
• 3-ethylphenol;
• 2-propylphenol;
• 2-sec-butylphenol;
• 2-tert-butylphenol;
• 3-tert-butylphenol;
• 2-methoxyphenol;
• 3-methoxyphenol;
• 2-ethoxyphenol;
• 3-ethoxyphenol;
• 2-chlorophenol;
• 3-chlorophenol;
• 2,3-dimethylphenol;
• 2,5-dimethylphenol;
• 2,6-dimethylphenol;
• 3,5-dimethylphenol;
• vanillin;
• 2,3-dichlorophenol;
• 2,6-dichlorophenol;
• 3,5-dichlorophenol;
• pyrogallol;
• 2,3,5-trimethylphenol;
• 2,3,6-trimethylphenol;
• 2,6-di-tert-butylphenol;
• 3,5-di-tert-butylphenol;
• 2,3,6-trichlorophenol;
• 1,2-dihydroxynaphthalene;
• 1,4-dihydroxynaphthalene;
• 1,5-dihydroxynaphthalene;
• 2,3-dihydroxynaphthalene;
• 2,6-dihydroxynaphthalene;
• 2,7-dihydroxynaphthalene;
• 4-methoxy-1-naphthol;
• 6-bromo-2-naphthol;
• 2-phenoxyphenol;
• 3-phenoxyphenol.

9. A process according to claim 1, **characterized in that** in a first step, the hydroxy function is protected by transforming it into a sulphonic ester.

10. A process according to claim 9, **characterized in that** preferred sulphonic ester groups are:
• tosylates (p-toluenesulphonates)-OSO₂-C₆H₄-CH₃;
• brosylates (p-bromobenzenesulphonates)-OSO₂-C₆H₄-Br;
• nosylates (p-nitrobenzenesulphonates)-OSO₂-C₆H₄-NO₂;
• mesylates (methanesulphonates)-OSO₂-CH₃;
• betylates (ammonioalkanesulphonates)-OSO₂-(CH₂)ₙNMe₃⁺ where n is in the range 0 to 6;
• triflates (trifluoromethanesulphonates) -OSO₂-CF₃;
• nonaflates (nonafluorobutanesulphonates)-OSO₂-C₄F₉;
• tresylates (2,2,2-trifluoroethanesulphonates)-OSO₂-CH₂-CF₃.

11. A process according to any one of claims 1 to 10, **characterized in that** the protected form of the phenolic compound has general formula (II): 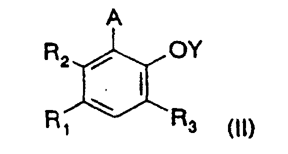 in which formula (II):
• Y represents the following group: 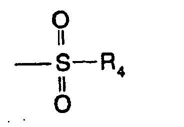 where R₄ represents a hydrocarbon radical containing 1 to 20 carbon atoms, more particularly:
• an alkyl radical containing 1 to 10 carbon atoms, preferably 1 to 4 carbon atoms, more preferably a methyl or ethyl radical, optionally carrying a halogen atom, a CF₃ group or an ammonium group N(R₅)₄, R₅, which may be identical or different, representing an alkyl radical containing 1 to 4 carbon atoms;
• a cycloalkyl radical containing 3 to 8 carbon atoms, preferably a cyclohexyl radical;
• an aryl radical containing 6 to 12 carbon atoms, preferably a phenyl radical optionally carrying an alkyl radical containing 1 to 10 carbon atoms, preferably 1 to 4 carbon atoms and more preferably a methyl or an ethyl radical, a halogen atom, a CF₃ group or a NO₂ group;
• a CX₃ group where X represents a fluorine, chlorine or bromine atom;
• a CF₂-CF₃ group;
• A, R₁, R₂ and R₃ have the meanings given above.

12. A process according to claim 11, **characterized in that** the protected phenol with formula (II) is obtained by reacting, in the presence of a base, a starting phenolic compound with formula (I) with a protecting agent with the following formula (III): 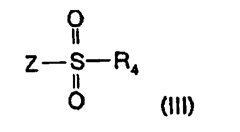 in which formula (III):
• R₄ has the meaning given above;
• Z represents:
• a hydroxy group or a halogen atom, preferably a chlorine or bromine atom;
• a -O-SO₂-R₄' group where R₄', which may be identical to or different from R₄, has the meaning given for R₄.

13. A process according to claim 12, **characterized in that** the protecting agent has formula (III) in which Z represents a chlorine or a bromine atom.

14. A process according to claim 12 or claim 13, **characterized in that** the protecting agent is selected from:
• triflic anhydride;
• methanesulphonyl chloride;
• benzenesulphonyl chloride;
• p-toluenesulphonyl chloride.

15. A process according to any one of claims 9 to 14, **characterized in that** a mineral or organic base is used.

16. A process according to claim 15, **characterized in that** the base used is an alkali metal hydroxide, preferably sodium or potassium hydroxide; an alkali metal carbonate, bicarbonate, phosphate, hydrogen phosphate, acetate or trifluoroacetate, preferably of sodium or potassium; or a tertiary amine.

17. A process according to claim 16, **characterized in that** the tertiary amine has general formula (IV):
N -(R₆)₃ (IV)
where:
• groups R₆, which may be identical or different, represent hydrocarbon residues containing 1 to 20 carbon atoms, such as alkyl, cycloalkyl, aryl or heterocyclic groups;
• two R₆ groups together with the nitrogen atom, form a heterocycle containing 4 to 5 atoms.

18. A process according to claim 17, **characterized in that** the amine is triethylamine, tri-n-propylamine, tri-n-butylamine, methyldibutylamine, methyldicyclohexylamine, ethyldiisopropylamine, N,N-diethylcyclohexylamine, dimethylamino-4-pyridine, N-methylpiperidine, N-ethylpiperidine, N-n-butylpiperidine, 1,2-dimethylpiperidine, N-methylpyrrolidone, or 1,2-dimethylpyrrolidine.

19. A process according to any one of claims 9 to 18, **characterized in that** the phenolic compound protection reaction is carried out in an organic, polar or apolar solvent, preferably an aliphatic or cycloaliphatic hydrocarbon which may or may not be halogenated; an ester; an ether or a nitrile.

20. A process according to any one of claims 9 to 19, **characterized in that** the quantity of base used, expressed as the ratio of the number of equivalents of OH to the number of moles of phenolic compound with formula (I), is in the range 0.5 to 3.0, preferably in the range 1.0 to 2.0.

21. A process according to any one of claims 9 to 20, **characterized in that** the quantity of protecting agent used, expressed as the ratio between the number of moles of protecting agent and the number of moles of starting phenolic compound, is in the range 1.0 to 1.5 and is preferably about 1.1.

22. A process according to any one of claims 9 to 21, **characterized in that** the temperature at which the operation for protecting the hydroxy group of the phenolic compound is carried out is between 0°C and ambient temperature (usually in the range 15°C to 25°C).

23. A process according to any one of claims 9 to 22, **characterized in that** the protected form of the phenolic compound is used as it is in the subsequent operation or is purified by recrystallisation from an organic solvent, preferably ethyl ether.

24. A process according to claim 1, **characterized in that** the protected form of the phenolic compound is reacted with an electrophilic reactant in an amidoalkylation reaction, a Friedel Crafts reaction such as acylation, sulphonylation or alkylation, an aminoalkylation reaction, a hydroxyalkylation reaction, a formylation reaction, a carboxylation reaction or a further electrophilic substitution reaction.

25. A process according to claim 24, **characterized in that** the protected phenolic compound, preferably with formula (II), is reacted, in the presence of a strong acid, with an amide or a carbamate and a carbonyl compound more particularly with general formula (V): 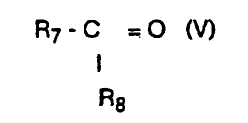 in which formula (V):
• R₇ represents a hydrogen atom;
• R₈ represents a hydrogen atom, a linear or branched alkyl radical containing 1 to 6 carbon atoms, a cycloalkyl radical containing 3 to 8 carbon atoms, an alkenyl radical containing 2 to 6 carbon atoms or a phenyl radical which may be substituted, or an electron-attracting group.

26. A process according to claim 25, **characterized in that** the electron-attracting group is:
• a -CHO group;
• a R₉-CO- or R₉-CO-(CH₂)ₘ- group where R₉ represents a linear or branched alkyl radical containing 1 to 6 carbon atoms and m represents a number from 1 to 3;
• a -COOR₁₀ group in which R₁₀ represents a hydrogen atom or a linear or branched alkyl radical containing 1 to 6 carbon atoms;
• a -CX₂H group where X represents a halogen atom, preferably a fluorine, chlorine or bromine atom;
• a -CX₃ group where X represents a halogen atom, preferably a fluorine, chlorine or bromine atom;
• a nitro group.

27. A process according to claim 25 or claim 26, **characterized in that** the carbonyl compound with formula (V) is formaldehyde or a formaldehyde generator; glyoxal, glyoxylic acid, bromal or fluoral; preferably paraformaldehyde.

28. A process according to claim 25, **characterized in that** the amide or carbamate used has the following formula (VI): 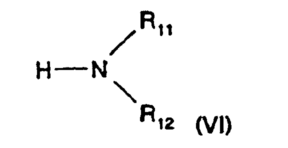 where:
• at least one of groups R₁₁ and R₁₂ represents a group: 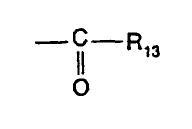 or 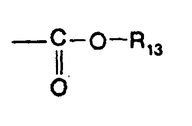
• groups R₁₁, R₁₂ and R₁₃, which may be identical or different, represent:
• a hydrogen atom;
• a linear or branched alkyl radical containing I to 12 carbon atoms, optionally carrying halogen atoms;
• a cycloalkyl radical containing 3 to 8 carbon atoms, preferably a cyclopentyl radical, a cyclohexyl radical or a cycloheptyl radical;
• a cycloalkylalkyl radical, preferably cyclopentylalkyl, cyclohexylalkyl, or cycloheptylalkyl where the alkyl portion contains 1 to 4 carbon atoms;
• an alkylaryl radical, preferably a phenylalkyl radical, where the alkyl portion contains 1 to 4 carbon atoms, preferably a benzyl radical;
• two groups R₁₁ and R₁₂, together with the two atoms carrying them, form a saturated, unsaturated or aromatic carbocylic cycle containing 3 to 8 carbon atoms.

29. a process according to claim 28, **characterized in that** the compound with formula (VI) is acetamide, chloracetamide, benzamide, or benzyl carbamate.

30. A process according to any one of claims 25 to 29, **characterized in that** the amidoalkylation reaction is carried out on the protected phenolic compound in the presence of a protonic acid with a pKa of 4.00 or less, preferably 3.00 or less.

31. A process according to claim 30, **characterized in that** the acid is selected from halogenated or non halogenated mineral oxyacids, preferably sulphuric acid; phosphoric acids; phosphonic acids; perhalogenated or non perhalogenated carboxylic acids; and halogenated or sulphonic acids.

32. A process according to any one of claims 25 to 31, **characterized in that** the reaction is carried out in an organic solvent, preferably an aliphatic carboxylic acid.

33. A process according to claim 32, **characterized in that** the aliphatic carboxylic acid is a saturated aliphatic carboxylic acid, preferably acetic acid, propionic acid, butyric acid, isobutyric acid, pentanoic acid, or 2-methylbutanoic acid.

34. A process according to any one of claims 25 to 33, **characterized in that** the ratio between the number of moles of carbonyl compound and the number of moles of protected phenolic compound is at least 1, preferably in the range 1.0 to 2.0.

35. A process according to any one of claims 25 to 34, **characterized in that** the ratio between the number of moles of amide or carbamate and the number of moles of carbonyl compound is in the range 1.0 to 3.0, preferably about 2.0.

36. A process according to any one of claims 25 to 35, **characterized in that** the quantity of strong acid used is such that the mole ratio of H⁺/protected phenolic compound is in the range 1.0 to 20, preferably in the range 2.0 to 3.0.

37. A process according to any one of claims 25 to 36, **characterized in that** the reaction is advantageously carried out between ambient temperature and 120°C, preferably in the range 40°C to 80°C.

38. A process according to any one of claims 1 to 37, **characterized in that** the amidoalkylated product where the hydroxy function is protected undergoes a treatment using a base employed in a quantity such that the pH is in the range 7 to 10 to cleave the sulphonyl group and liberate the hydroxy group to obtain an amidoalkylated phenolic compound where the amidoalkyl group is in the position para to the electron-donating group.

39. A process according to any one of claims 1 to 38, **characterized in that** the amidoalkylated group where the hydroxy function is protected is reacted with an acidic solution, preferably a hydrochloric acid solution, to obtain a protected and aminoalkylated phenolic compound where the aminoalkyl group is in the position para to the electron-donating group.

40. A process according to claim 24, **characterized in that** the protected phenolic compound preferably having formula (II) is reacted with an electrophilic reactant, in the presence of a Friedel-Crafts catalyst.

41. A process according to claim 40, **characterized in that** the electrophilic reactants used are carboxylic acids and their halide or anhydride derivatives, preferably anhydride; sulphonyl or aminosulphonyl halides or anhydrides; or halide or alcohol type alkylating agents.

42. A process according to claim 40 or claim 41, **characterized in that** the acylation or sulphonylation reactants more particularly have the following formulae: 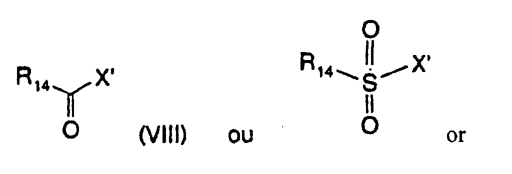
• in which formulae (VIII) or (IX):
• R₁₄ represents a linear or branched, saturated or unsaturated aliphatic radical containing 1 to 24 carbon atoms; a saturated, unsaturated or aromatic, monocyclic or polycyclic cycloaliphatic radical containing 4 to 12 carbon atoms; or a linear or branched, saturated or unsaturated aliphatic radical carrying a cyclic substituent;
• X' represents a halogen atom, preferably a chlorine or bromine atom;
• in formula (VIII):
• X' represents a -O-CO-R₁₅ radical where R₁₅, which may be identical to or different from R₁₄, has the same meaning as R₁₄;
• in formula (IX):
• X' represents a -O-SO₂-R₁₅ radical where R₁₅, which may be identical to or different from R₁₄, has the same meaning as R₁₄;
• R₁₄ represents:
• an R₁₆-O- alkoxy radical where R₁₆ has the same meaning as R₁₄;
• an amino group (R₁₇)(R₁₈)-N- where R₁₇, which may be identical to or different from R₁₈, has the same meaning as R₁₄.

43. A process according to claim 40 or claim 41, **characterized in that** the alkylation reactant has general formula (X):
R₁₈-X (X)
in which formula (X):
• X represents a hydroxy group or a halogen atom; said hydroxy group or halogen atom must not be in the vinyl or alkynyl position;
• R₁₈, which may be substituted, represents a linear or branched, saturated or unsaturated acyclic aliphatic radical; a saturated or unsaturated, monocyclic or polycyclic cycloaliphatic radical ; or a linear or branched, saturated or unsaturated aliphatic radical carrying a cyclic substituent.

44. A process according to claim 24, **characterized in that** the protected phenolic compound preferably with formula (II) is reacted with a carbonyl compound, in the presence of a secondary amine.

45. A process according to claim 44, **characterized in that** the carbonyl compound has general formula (XI): 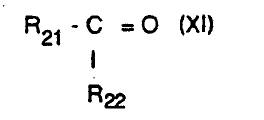 in which formula (XI):
• R₂₁ represents a hydrogen atom, a linear or branched alkyl radical containing 1 to 6 carbon atoms, an alkenyl radical containing 2 to 6 carbon atoms, or a phenyl radical which may be substituted;
• R₂₂ can be equal to R₂₁ or it can represent an electron-attracting group.

46. A process according to claim 44, **characterized in that** the secondary amine has the following formula (XII): 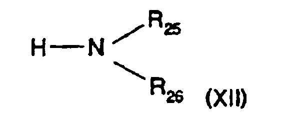 where:
• R₂₅ and R₂₆, which may be identical or different, represent:
• a linear or alkyl radical containing 1 to 12 carbon atoms, a secondary alkyl radical containing 3 to 12 carbon atoms or a tertiary alkyl radical containing 4 to 12 carbon atoms; the alkyl radicals may contain one or two ether functions -0- or hydroxy functions, tertiary amine, carboxylic acid, carboxylic acid ester functions;
• a phenylalkyl, cyclohexylalkyl, cycloheptylalkyl or cyclopentylalkyl radical wherein the alkyl portion contains 1 to 4 carbon atoms;
• a hydrogen atom;
• a phenyl radical, a cyclohexyl radical, a cycloheptyl radical or a cyclopentyl radical;
• R₂₅ or R₂₆, together with the nitrogen atom, form a heterocycle which may be saturated or contain one or more double bonds, optionally substituted with one or more alkyl radicals containing 1 to 4 carbon atoms;
• R₂₅ and R₂₆ together with the nitrogen atom and with one or more nitrogen and/or oxygen and/or sulphur atoms form a saturated or unsaturated heterocycle optionally substituted by one or more alkyl radicals containing 1 to 4 carbon atoms;
• R₂₅ and R₂₆ together with the nitrogen atom form an unsaturated heterocycle optionally substituted by one or two methyl or ethyl groups;
• R₂₅ and R₂₆ together with the nitrogen atom and optionally with one or more nitrogen and/or oxygen and/or sulphur atoms form a saturated or unsaturated polycyclic compound optionally substituted by one or more alkyl radicals containing 1 to 4 carbon atoms.

47. A process according to claim 44, **characterized in that** the carbonyl compound is reacted then the secondary amine to form the hemiaminal reactant, then the protected phenolic compound and the acid catalyst.

48. A process according to claim 24, **characterized in that** the protected phenolic compound preferably with formula (II) is reacted with a carbonyl compound in the presence of a Brönsted or a zeolite type catalyst.

49. A process according to claim 48, **characterized in that** the carbonyl compound has general formula (XI) as defined in claim 45.

50. A process according to claim 24, **characterized in that** the protected phenolic compound preferably with formula (II) is reacted with chloral or hexamethylenetetramine.

51. A process according to claim 50, **characterized in that** the reaction is carried out in an acid, preferably trifluoroacetic acid, sulphuric acid, hydrofluoric acid or boric acid.

52. A process according to any one of claims 40 to 51, **characterized in that** the electrophilic substitution step is followed by a hydroxy group de-protecting step, preferably employing the implementation described in claim 38.
